# EUROPEAN PATENT APPLICATION

(11) **EP 4 681 625 A2**
(43) Date of publication of application: **21.01.2026**
(21) Application number: 25207540.3
(22) Date of filing: 20.03.2024
(51) Int. Cl.: A61B 5/022

(54) **AIR-PRESSURE CUFFS**

(62) Divisional of application: 24714440.5
(71) Applicant: Rodiera Olive, José Javier, 08022 Barcelona (ES)
(72) Inventor: Rodiera Olive, José Javier, 08022 Barcelona (ES)
(74) Representative: ZBM Patents - Zea, Barlocci & Markvardsen

(57) **Abstract**

Interface medical systems are provided which permit two different medical/healthcare monitors to operate using a same air-pressure cuff through an interface arrangement intermediating between the cuff and one of the monitors from two-ways air transmission perspective, i.e., from one of the medical/healthcare monitors to cuff and from cuff to said one of the medical/healthcare monitors. Memory equipped pressure cuffs are also disclosed providing storing functionalities significantly improving prior art pressure cuffs. Flex-connector pressure cuffs are provided as well including a versatile connector between inflatable bag and air tube forming the pressure cuff. Add-sensor pressure cuffs are also disclosed including additional non-pressure-based sensors providing powerful capabilities of measuring different types of medical conditions simultaneously, without the need of manipulating different devices nor disturbing the patient with different operations to measure different medical conditions. Methods and computer programs for controlling at least some of said systems and pressure cuffs are also provided.

## Description

The present disclosure relates to medical systems comprising an air-pressure cuff and a medical monitor, to methods and computer programs of controlling such medical systems, and to controllers for controlling such medical systems.

The present disclosure further relates to air-pressure cuffs for non-invasively measuring blood pressure of a patient, and to methods and computer programs of controlling such air-pressure cuffs.

### BACKGROUND

Blood pressure measurement is a priority recommendation in surgical procedures to which patients are subjected. Operating/surgical rooms are equipped with at least one multiparameter monitor that, among other things, allows non-invasive blood pressure measurement through a cuff. This measurement can be carried out automatically at programmed intervals or manually when the healthcare professional considers it appropriate. On the other hand, in the operating room there are other complementary monitors that measure other parameters such as consciousness, breathing, analgesia or neuromuscular transmission. These various monitors have wires, tubes, peripherals, etc. associated thereto that may be disturb medical or surgical staff, which can be numerous, during performance of surgical operations or medical treatments. It is currently a concern to reduce the number of and occupied space by medical/surgical devices and surrounding components potentially distorting activity of medical/surgical professionals. It is also a concern to reduce costs associated to not strictly necessary medical/surgical components in medical/surgical areas, as well as to reduce environmental impact or contamination.

Pressure cuffs are commonly used in hospitals or medical centres, with many equipment units (monitors) and areas where patients' pressure is measured. Patients may move through the hospital between different areas. For example, it is common that a patient who has entered the hospital through emergencies goes to hospitalization floor, then goes to operating/surgical room, then goes to the ICU or resuscitation unit and then returns to hospitalization floor.

Pressure cuffs also deteriorate with use so that they can have a predefined useful life depending on use. It is sometimes difficult to detect when a pressure cuff must be removed or replaced by another. In the case of single-use or single-patient disposable cuffs, they can sometimes be exchanged between patients or may be used more than once inadvertently.

Also, quick and easy access to certain data from patients' clinical history and having the most relevant clinical information and data of patients in an easy and accessible way can be very important, mainly in some critical situations or even in everyday situations.

Pressure cuffs normally incorporate a tube portion of approximately between 20 - 30 cm, which makes relatively easy the connection of the cuff with tube part from medical monitor. In the case of hospital monitors, whole tube length can vary, but it may be generally of between 2 and 3 meters. The aforementioned (20 - 30 cm of) tube portion makes easier to change/replace the cuff on corresponding patient without the need to waste the 2 - 3 meters of tubing. It also allows patient mobility without having to drag the entire (2 - 3 meters of) tube or the need to remove the cuff from patient to move from one place to another in, e.g., the hospital. However, the (20 - 30 cm of) tube portion may be disturbing or annoying in some circumstances.

An object of this disclosure is to provide new pressure cuffs for non-invasively measuring at least blood pressure, systems including such pressure cuffs and methods of controlling them with the aim of resolving aforementioned drawbacks and/or improving prior art pressure cuffs, as well as devices, systems and methods using or including such prior art pressure cuffs.

### SUMMARY

In an aspect, medical systems are provided comprising an air-pressure cuff and a medical monitor, the air-pressure cuff having an inflatable bag installable in predefined disposition around a patient's limb, and the medical monitor being configured to perform non-invasive blood pressure measurement(s) on the patient through the air-pressure cuff. The medical monitor comprises an interface arrangement configured to implement an air connection between the medical monitor and a healthcare monitor different from the medical monitor, and to operate an air flow regulator to keep the air connection opened or closed. The interface arrangement is configured to operate an air flow detector to detect start of non-invasive blood pressure measurement from the healthcare monitor and, in response to said detection, to operate the air flow regulator to keep the air connection opened in such a manner that the started non-invasive blood pressure measurement is performed by the healthcare monitor through the air-pressure cuff of the medical system instead of using an air-pressure cuff directly associated to the healthcare monitor. These medical systems may also be denominated "interface medical systems" herein.

The proposed interfacing between healthcare monitor and pressure-cuff "outsider" with respect to said monitor provides different advantages. In scenarios where healthcare monitor and medical monitor have distinct functions but one of them requires using a pressure-cuff in both monitors, the pressure-cuff directly associated to the healthcare monitor may be avoided. This way, less medical/surgical components may be present in corresponding medical/surgical area, so potential disturbing due to existence of many medical/surgical components is alleviated. Also, efficiency is provided in the sense that no substitution of one pressure-cuff by another on the patient is required, since both healthcare monitor and medical monitor may use same pressure-cuff. Furthermore, if healthcare monitor has primary role and medical monitor has secondary role, functions of the latter requiring pressure-cuff may be attributed uniquely to the former, so duplication of functions may be minimized or at least attenuated. Besides, it is common that primary monitor is connected to an electronic data collection system and the secondary monitor is not, so said diverting of functions from secondary monitor to primary monitor may allow collecting data impossible or difficult to collect without such a functional diverting or substitution.

In interface medical systems of example, the inflatable bag may have non-invasive electrodes electrically connected or connectable with the medical monitor and arranged in/on/at the inflatable bag in such a manner that, when (the inflatable bag is) installed in the predefined disposition, the non-invasive electrodes result/are placed onto a target muscle's nerve of the patient's limb. Furthermore, the medical monitor may be configured to electro-stimulate the target muscle's nerve through the non-invasive electrodes to provoke a muscular response by the target muscle to said electro-stimulation, and to determine a neuromuscular condition of the patient depending on air pressure variations in the inflatable bag caused by said muscular response. Such a neuromuscular condition may include a neuromuscular blockade state of the patient. In this scenario, if medical monitor has secondary role with respect to healthcare monitor, duplicated functions may be diverted from medical monitor to healthcare monitor and medical monitor may be exclusively or almost exclusively dedicated to neuromuscular functions, which are not normally available in primary monitors.

In exemplary interface medical systems, the interface arrangement may be configured to operate the air flow regulator to keep the air connection opened by default to give priority to the healthcare monitor over the medical monitor. This feature may be specially advantageous in scenarios where healthcare monitor has primary role and medical monitor has secondary role.

In implementations of interface medical systems, the interface arrangement may be configured to operate the air flow regulator to keep the air connection closed during performance of non-invasive blood pressure measurement by the medical monitor through its pressure cuff and, optionally, upon completion of said measurement, air flow regulator may be operated to open air connection to return it to open default state. This operation mode based on not interrupting measurement in progress by medical monitor upon detection of measurement start by healthcare monitor may be denominated "relaxed priority policy." That is, healthcare monitor has priority over medical monitor but not so strictly as to interrupt measurement in progress by medical monitor.

In interface medical systems according to examples, the interface arrangement may be configured to operate the air flow regulator to open the air connection upon detection of start of non-invasive blood pressure measurement from the healthcare monitor, irrespective of whether non-invasive blood pressure measurement by the medical monitor is in progress or not. This operation mode based on interrupting measurement in progress by medical monitor upon detection of measurement start by healthcare monitor may be denominated "strict priority policy." That is, healthcare monitor has priority over medical monitor so strictly that measurement in progress by medical monitor is interrupted.

In some examples, the air connection implemented by the interface arrangement may include a pneumatic air connection, and/or the air flow regulator may include a valve operable to open and close the air connection, and/or the air flow detector may include an air pressure sensor. The valve may include, e.g., a three-way valve. The air pressure sensor may include, e.g., a piezoresistive air pressure sensor, a capacitive air pressure sensor, a MEMS-based air pressure sensor or any combination thereof.

In interface medical systems according to some implementations, the interface arrangement may be configured to detect start of non-invasive blood pressure measurement from the healthcare monitor through the air pressure sensor depending on whether a pressure detected by the air pressure sensor is above or below predefined pressure threshold or within or outside predefined pressure range.

Air-pressure cuffs included in interface medical systems may be similar to any of the memory pressure cuffs or flex-connector pressure cuffs or add-sensor pressure cuffs disclosed herein or may have any of the features thereof.

Healthcare systems may also be provided including any of the interface medical systems disclosed herein and the healthcare monitor. Medical or surgical rooms including any of these healthcare systems may also be provided. Medical or surgical vehicles including any of these healthcare systems may be provided as well.

In a further aspect, methods are provided of controlling any of the interface medical systems disclosed in present disclosure. These methods (also denominated "interface control methods" herein) comprise operating the air flow detector to detect start of non-invasive blood pressure measurement from the healthcare monitor and, in response to said detection, operating the air flow regulator to keep the air connection opened. With such a keeping of the air connection opened, the started non-invasive blood pressure measurement is performed by the healthcare monitor through the air-pressure cuff of the medical system instead of using an air-pressure cuff directly associated to the healthcare monitor. These interface control methods are performable (or suitable to be performed) by interface medical systems proposed herein, so same or similar functional principles and advantages as the ones described with reference to interface medical systems may be attributed to such interface control methods.

In a still further aspect, computer programs are provided comprising program instructions for causing a system or computer system or controller to perform interface control methods aimed at controlling any of the interface medical systems disclosed in present disclosure. These computer programs (also denominated "interface control computer programs" herein) may be embodied on a storage medium and/or carried on a carrier signal. Interface control computer programs are suitable or configured to perform any of the interface control methods proposed herein, so same or similar functional fundamentals and advantages as the ones described with reference to interface control methods may be attributed to such interface control computer programs.

In a yet further aspect, controllers are provided for controlling any of the interface medical systems disclosed in present disclosure, said controllers comprising a memory and a processor, embodying instructions stored in the memory and executable by the processor, and the instructions comprising functionality or functionalities to execute interface control methods such as those described in other parts of the disclosure. In alternative implementations, such controllers (also denominated "interface controllers" herein) include a detector module and an operator module. The detector module is configured to operate the air flow detector to detect start of non-invasive blood pressure measurement from the healthcare monitor and, in response to said detection, the operator module is configured to operate the air flow regulator to keep the air connection opened. With such a keeping of the air connection opened, the started non-invasive blood pressure measurement is performed by the healthcare monitor through the air-pressure cuff of the medical system instead of using an air-pressure cuff directly associated to the healthcare monitor. These interface controllers are suitable or configured to perform any of the interface control methods proposed herein, so same or similar functional principles and advantages as the ones described with reference to interface control methods may be attributed to such interface controllers.

In another aspect, air-pressure cuffs are provided comprising an inflatable bag, a connector arrangement, a memory unit, data-comm unit (or data-communication unit) and a controller module. In these air-pressure cuffs (also denominated "memory pressure cuffs" herein), the inflatable bag is configured to be installed in predefined disposition around a patient's limb and to be inflated to experience air pressure variations in its inside due to interaction with the patient's limb. The connector arrangement includes an air tube configured to transmit air from a medical monitor to the inflatable bag to inflate it and to transmit air pressure variations from the inflatable bag to the medical monitor, in such a manner that patient's blood pressure is non-invasively measurable by the medical monitor depending on said air pressure variations. The memory unit is configured to store data. The data-comm unit is configured to implement data connection between the medical monitor, or another type of computing device, and the memory unit. The controller module is configured to operate the data-comm unit to implement the data connection and to operate the memory unit to exchange data between the medical monitor, or the other type of computing device, and the memory unit through the implemented data connection.

With such memory (or mem-equipped) pressure cuffs, every patient may have its cuff of said type associated therewith and may go through different areas in hospital or even to/from other medical centres with relevant data stored in its associated cuff. Patient's memory pressure cuff may be connected to and disconnected from different medical devices/monitors which, when connected, may use the data stored in the cuff. This may permit, e.g., quick and easy access to data of the patient (e.g., his/her clinical history), which may be very important in some critical situations or even in everyday situations.

Since pressure cuffs may deteriorate with use, mem-equipped pressure cuffs according to present disclosure may also provide advantages regarding whether maximum use of the cuff has been exceeded or, otherwise, it is still supposed to be in good conditions to be used. In current medical practice, it is sometimes difficult to detect when a pressure cuff must be removed or replaced by another, and said difficulty is eliminated or attenuated by mem-equipped pressure cuffs disclosed herein. Data about use of mem-equipped pressure cuff may be stored in its memory unit and accordingly used to check its validity for use. In the case of single-use or single-patient disposable cuffs, something similar may occur. They can sometimes be exchanged between patients or may be used more than once inadvertently. This problem may also be solved by using mem-equipped pressure cuffs according to present disclosure based on controlling use of the mem-equipped pressure cuff by storing use data in its memory unit and using it to control that appropriate use of the cuff is made.

In exemplary memory pressure cuffs, the connector arrangement may comprise a wired data connection integrated with or within the air tube, the wired data connection being operable by the controller module to exchange data between the medical monitor and the memory unit. This feature may also be beneficial in the sense that no added disturbing tubes or cables are needed because the wired data connection between the cuff and the monitor is "hidden" within the already existing air tube. Data exchange between mem-equipped cuff and monitor may thus be performed through the wired data connection with no added disturbing.

In memory pressure cuffs of example, the data-comm unit may comprise a physical connection unit operable by the controller module to establish physical data connection with the medical monitor, or the other type of computing device, and to exchange data through said physical data connection between the medical monitor, or the other type of computing device, and the memory unit. Such a physical connection unit, which may be, e.g., an USB-based connection unit, may be very useful to exchange data between the memory unit and another computing device different from the medical monitor. This physical connection unit may be an alternative to the wired data connection or an additional means to exchange data with the medical monitor. Flexibility is thus added by this feature to memory pressure cuffs according to present disclosure.

In memory pressure cuffs according to implementations, the data-comm unit may comprise a wireless unit operable by the controller module to establish wireless data connection with the medical monitor, or the other type of computing device, and to exchange data through said wireless data connection between the medical monitor, or the other type of computing device, and the memory unit. Same or similar benefits as the ones provided by the physical connection unit may be attributed to these memory pressure cuffs with wireless unit. Additionally, the wireless unit and its operation in the context of memory pressure cuffs according to present disclosure do not add disturbing cables/wires between the cuff and the monitor. Flexibility with no disturbing conditions is thus added by this feature to memory pressure cuffs according to present disclosure.

In some configurations, the wireless data connection implementable through/by the wireless unit may be a Radio Frequency connection, an NFC connection, a wifi connection, a bluetooth connection, a RFID connection, any other type of wireless data connection or any combination thereof.

In examples of memory pressure cuffs, the inflatable bag may have non-invasive electrodes arranged in/on/at the inflatable bag in such a manner that, when (the inflatable bag is) installed in the predefined disposition, the non-invasive electrodes result placed onto a target muscle's nerve of the patient's limb. In turn, the connector arrangement may include an electro-connection configured to transmit electro-stimulation signals from the medical monitor to the non-invasive electrodes to electro-stimulate the target muscle's nerve of the patient's limb. Said electro-stimulation is such that a muscular response by the target muscle is provoked thereby causing air pressure variations in the inflatable bag that are usable by the medical monitor to determine a medical condition of the patient depending on said air pressure variations. Such a medical condition may be a neuromuscular condition and, in particular, a neuromuscular blockade status of the patient. Very powerful mem-equipped pressure cuffs with neuromuscular detection functionalities may thus be provided thanks to this feature regarding electro-stimulation electrodes and their use to categorize patients from neuromuscular perspective.

In exemplary memory pressure cuffs, the memory unit may permanently store invariable data of the air-pressure cuff. Said invariable data may include, e.g., manufacturing lot, or serial number, or manufacturer, or manufacturing date, or maximum number of uses, or expiration date, or indicator of disposable cuff, or useful life of the cuff, or electrodes impedance (if any), etc.

In some memory pressure cuffs, the controller module may be configured to automatically update variable data of the air-pressure cuff in the memory unit. Such updatable variable data may include, e.g., number of performed uses, or remaining useful life, or ID of patient using the cuff, or ID of last patient used the cuff, etc.

In implementations of memory pressure cuffs, the controller module may be configured to store and/or update patient-related data in the memory unit. Said patient-related data may be either obtained locally at the air-pressure cuff or received from the medical monitor, or the other type of computing device, through data connection implemented by the data-comm unit. This storable and/or updatable patient-related data may include, e.g., last arterial pressure measurements, or last neuromuscular-related measurements, or allergies, or clinical history data, or current medication, or past medication, or patient's route through hospital between medical areas with dates and times, etc. or any combination thereof.

In memory pressure cuffs according to examples, the controller module may be configured to automatically dump data stored in the memory unit to the medical monitor, or the other type of computing device. Said cuff-to-monitor/computer dump may be triggered upon detection of a cuff-to-monitor dump request or upon detection of effective connection between the air-pressure cuff and the medical monitor, or the other type of computing device. With such a cuff-to-monitor dump function, one or another medical monitor (or computing system) may operate with data from the cuff associated with particular patient very easily and quickly, irrespective of where the patient is at each time in the hospital or medical centre. By dumping data from (mem-equipped pressure) cuff to medical monitor (or computing system), the latter becomes automatically operative to function dealing with data from the cuff and, therefore, related to the patient wearing the (mem-equipped pressure) cuff.

In memory pressure cuffs of example, the controller module may be configured to automatically dump data stored in the medical monitor, or the other type of computing device, to the memory unit. Said monitor/computer-to-cuff dump may be triggered upon detection of monitor-to-cuff dump request or upon detection of effective connection between the air-pressure cuff and the medical monitor, or the other type of computing device. Such a monitor-to-cuff dump function may permit download data from medical monitor to the (mem-equipped pressure) cuff very easily and quickly, such that patient associated to the cuff may go to one or another area of the hospital or medical centre with his/her data stored in the (mem-equipped pressure) cuff.

In both data dump approaches, cuff-to-monitor dump and monitor-to-cuff dump, the controller module may be configured to select the data to be dumped by patient's ID.

Memory (or mem-equipped) pressure cuffs may be similar to any of the flex-connector pressure cuffs or add-sensor pressure cuffs or air-pressure cuffs in interface medical systems disclosed herein or may have any of the features thereof.

Pressure measuring systems (or simply measuring systems) may also be provided including any of the memory pressure cuffs described herein and the medical monitor, or the other type of computing device. Medical or surgical rooms including any of said pressure measuring systems may also be provided. Medical or surgical vehicles including any of said pressure measuring systems may be provided as well.

In a further other aspect, methods are provided of controlling any of the memory pressure cuffs disclosed in present disclosure. Such methods (also denominated "memory control methods" herein) comprise operating, by the controller module, the data-comm unit to implement data connection between the medical monitor, or the other type of computing device, and the memory unit. These memory control methods further comprise operating, by the controller module, the memory unit to exchange data between the medical monitor, or the other type of computing device, and the memory unit through the implemented data connection. These memory control methods are performable (or suitable to be performed) by/at memory (or mem-equipped) pressure cuffs proposed herein, so same or similar functional principles and advantages as the ones described with reference to memory pressure cuffs may be attributed to such memory control methods.

In a still further other aspect, computer programs are provided comprising program instructions for causing a system or computer system or controller to perform memory control methods aimed at controlling any of the memory pressure cuffs disclosed in present disclosure. These computer programs (also denominated "memory control computer programs" herein) may be embodied on a storage medium and/or carried on a carrier signal. Memory control computer programs are suitable or configured to perform any of the memory control methods proposed herein, so same or similar functional fundamentals and advantages as the ones described with reference to memory control methods may be attributed to such memory control computer programs.

In a furthermore aspect, air-pressure cuffs are provided comprising an inflatable bag including a bag-side connector and an air tube including at one end thereof a tube-side connector, said bag-side and tube-side connectors being removably couplable with each other in tight-fitting or press-fitting insertable manner. In these air-pressure cuffs (also denominated flex-connector pressure cuffs herein) the inflatable bag is configured to be installed in predefined disposition around a patient's limb and to be inflated to experience air pressure variations in its inside due to interaction with the patient's limb. Besides, the air tube is configured to transmit air from medical monitor to the inflatable bag to inflate it and to transmit air pressure variations from the inflatable bag to the medical monitor, in such a manner that patient's blood pressure is non-invasively measurable by the medical monitor depending on said air pressure variations.

These flex-connector pressure cuffs may avoid disturbing situations due to permanent presence of air tube or portion thereof. Since entire air tube may be easily connected to and disconnected from the inflatable bag by duly handling the bag-side and tube-side connectors, no disturbing tube portion remains hanging from the inflatable bag when not used. Thus, (flex-connector) pressure cuff may be permanently mounted around patient's arm in such a way that a variety of functionalities associated to the cuff may be easily and quickly enabled and executed. Movement of patients through whole hospital or medical centre carrying their associated cuff is also facilitated, along with connection and disconnection of the cuff to/from different medical monitors as needed. This flexibility makes the proposed flex-connector pressure cuffs very valuable. Additionally, flex-connector pressure cuffs are more environmentally friendly than prior art pressure cuffs with non-recyclable tube portion which is avoided in flex-connector pressure cuffs.

In some examples of flex-connector pressure cuffs, one of the bag-side and tube-side connectors may be a male connector and the other of the bag-side and tube-side connectors may be a female connector. This feature may make coupling and decoupling between inflatable bag and air tube very easy and quick.

In flex-connector pressure cuffs of example, the bag-side and tube-side connectors may be rounded-shaped in such a manner that, when the bag-side and tube-side connectors are coupled with each other, the bag-side and tube-side connectors are rotatable with respect to each other. This feature may add particularly interesting benefits from patient's movement perspective when the bag-side and tube-side connectors are coupled with each other, since free rotation between them may avoid restrictions on free of movement by patient wearing the cuff and even by medical staff assisting the patient.

In implementations of flex-connector pressure cuffs, the bag-side and tube-side connectors may be cylinder-shaped in such a manner that, when the bag-side and tube-side connectors are coupled with each other, the bag-side and tube-side connectors are rotatable with respect to each other.

In exemplary flex-connector pressure cuffs, the bag-side and tube-side connectors may include a click-fit coupling mechanism. In particular examples, said click-fit coupling mechanism may be based on a protruding part at one of the bag-side and tube-side connectors and a recessed part at the other of the bag-side and tube-side connectors in such a manner that coupling of the bag-side and tube-side connectors with each other causes the protruding part to click-fit the recessed part. Such a click-fit coupling mechanism may improve balance between tightness of the connection between the bag-side and tube-side connectors and rotatability and, therefore, free relative movement between them, as well as ease of coupling.

In flex-connector pressure cuffs according to examples, the air tube may further include one or more electro-lines along whole length of the air tube, each of the electro-lines being configured to transmit electro-signals.

In implementations of flex-connector pressure cuffs, the bag-side and tube-side connectors may implement, when the (bag-side and tube-side) connectors are coupled with each other, an electro-connection between the inflatable bag and the air tube according to a track/pin-based approach. This approach may be based on that each of the electro-lines terminates at the tube-side connector with a connection pin or track arranged to, when the (bag-side and tube-side) connectors are coupled with each other, electrically contact an associated connection pin or track at the bag-side connector.

In examples according to the track/pin-based approach, the electro-connection may be fully based on connection tracks at both the bag-side and tube-side connectors, with said connection tracks arranged in following manner. Each of the bag-side and tube-side connectors may have its respective connection tracks concentrically disposed in such a manner that, when the (bag-side and tube-side) connectors are coupled with each other, each of the connection tracks at the bag-side connector permanently contacts its associated connection track at the tube-side connector, irrespective of any rotational movement between the bag-side and tube-side connectors.

In further examples according to the track/pin-based approach, the electro-connection may be based on connection tracks at the bag-side connector and connection pins at the tube-side connector, with said connection tracks and pins arranged in following manner. The bag-side connector may have its connection tracks concentrically arranged in such a manner that, when the (bag-side and tube-side) connectors are coupled with each other, each of the connection tracks at the bag-side connector permanently contacts its associated connection pin (or pins) at the tube-side connector, irrespective of any rotational movement between the bag-side and tube-side connectors.

In still further examples according to the track/pin-based approach, the electro-connection may be based on connection pins at the bag-side connector and connection tracks at the tube-side connector, with said connection tracks and pins arranged in following manner. The tube-side connector may have its connection concentrically arranged in such a manner that, when the (bag-side and tube-side) connectors are coupled with each other, each of the connection tracks at the tube-side connector permanently contacts its associated connection pin (or pins) at the bag-side connector, irrespective of any rotational movement between the bag-side and tube-side connectors.

Above features regarding electro-connections and their implementation in the bag-side and tube-side connectors according to the track/pin-based approaches, provide benefits in terms of good balance between multifunctionality and flexibility. Multifunctional flex-connector pressure cuffs may thus be provided, with both air-based and electro-signal-based functions, while keeping flexibility, ease and quickness of coupling commented in other parts of the disclosure.

In examples of flex-connector pressure cuffs, the inflatable bag may have non-invasive electrodes arranged in/on/at the inflatable bag in such a manner that, when (the inflatable bag is) in the predefined disposition, the non-invasive electrodes result placed onto a target muscle's nerve of the patient's limb. In same examples, each of the non-invasive electrodes may be electrically connected with respectively associated connection pin or track at the bag-side connector. Still in same examples, at least some of the electro-lines may be configured to transmit electro-stimulation signals from the medical monitor to the non-invasive electrodes to electro-stimulate the target muscle's nerve of the patient's limb. Such an electro-stimulation may be such that muscular response by the target muscle is incited, thereby causing air pressure variations in the inflatable bag to be used by the medical monitor to determine a medical condition of the patient depending on said air pressure variations. Such a medical condition of the patient determinable by the medical monitor may be a neuromuscular condition such as, e.g., a neuromuscular blockade status of the patient.

The above features regarding electro-stimulation to provoke muscle response and air pressure variations due to said response, and determination of medical condition based on said pressure variations further expand the multifunctional power of flex-connector pressure cuffs. Hence, with such characteristics, even more powerful pressure cuffs with good balance between flexibility, ease and quickness of coupling and multifunctionality may be provided.

Flex-connector pressure cuffs according to some examples may further comprise a memory unit and a controller module configured to operate the memory unit to store and/or update data in the memory unit, and/or to exchange data between the medical monitor and the memory unit through at least some of the electro-lines. The data storable and/or updatable in the memory unit may be data generated at the air-pressure cuff itself or received from the medical monitor through at least some of the electro-lines. These data storing and exchange features expand still even more the multifunctional power of flex-connector pressure cuffs. Hence, with such features, yet even more powerful pressure cuffs with good balance between flexibility, ease and quickness of coupling and great multifunctionality may be provided.

Flex-connector pressure cuffs may be similar to any of the memory (or mem-equipped) pressure cuffs or add-sensor pressure cuffs or air-pressure cuffs in interface medical systems disclosed herein or may have any of the features thereof.

Pressure measuring systems (or simply measuring systems) may also be provided including any of the flex-connector pressure cuffs described herein and the medical monitor. Medical or surgical rooms including any of said pressure measuring systems may also be provided. Medical or surgical vehicles including any of said pressure measuring systems may be provided as well.

In an additional aspect, air-pressure cuffs are provided comprising an inflatable bag, an air tube and one or more non-invasive non-pressure-based sensors. In these air-pressure cuffs (also denominated "add-sensor pressure cuffs" or "pressure cuffs with added sensors" herein), the inflatable bag is configured to be installed in predefined disposition around a patient's limb and to be inflated to experience air pressure variations in its inside due to interaction with the patient's limb. The air tube is configured to transmit air from a healthcare monitor to the inflatable bag to inflate it and to transmit air pressure variations from the inflatable bag to the healthcare monitor, in such a manner that patient's blood pressure is non-invasively measurable by the healthcare monitor depending on said air pressure variations. The one or more non-invasive non-pressure-based sensors are arranged in/on/at the inflatable bag in such a manner that, when (the inflatable bag is) installed in the predefined disposition, the one or more non-invasive non-pressure-based sensors contact patient's skin.

These add-sensor pressure cuffs (or pressure cuffs with added sensors) may thus provide multifunctional advantages from measuring perspective. Not only pressure-based measurements are provided by add-sensor pressure cuffs but, as explained in other parts of the disclosure, they may also provide measurements of temperature, oxygen saturation, ECG, etc. Such multifunctional (add-sensor) pressure cuffs may be very valuable in hospitals or medical centres in general to obtain different measurements from patients without the need to change of measurer every time a different medical parameter has to be measured.

In exemplary add-sensor pressure cuffs, the one or more non-invasive non-pressure-based sensors may include a non-invasive temperature sensor which may, in some examples, be arranged in/on/at the inflatable bag in such a manner that, when (the inflatable bag is) installed in the predefined disposition, the non-invasive temperature sensor results positioned onto brachial artery of the patient's limb. This arrangement of the non-invasive temperature sensor may provide especially accurate temperature measurements.

In add-sensor pressure cuffs according to some implementations, a surface of the inflatable bag in/on/at which the non-invasive temperature sensor is arranged may include thermal insulating material to avoid or minimize distortion of patient's temperature due to environmental conditions. This feature may permit multifunctional add-sensor pressure cuffs to provide even more accurate and reliable temperature measurements.

In implementations, the non-invasive temperature sensor may be based on infrared LED technology or PT100 probe technology or K type probe technology or any combination thereof.

In examples of add-sensor pressure cuffs, the one or more non-invasive non-pressure-based sensors may include a non-invasive oxygen saturation sensor or pulse oximeter which may, in some configurations, be arranged in/on/at the inflatable bag in such a manner that, when (the inflatable bag is) installed in the predefined disposition, the non-invasive oxygen saturation sensor results positioned with sensing surface or surfaces thereof fully contacting patient's skin. These features regarding oxygen saturation sensor and its arrangement in/on/at the inflatable bag enable such add-sensor pressure cuffs to provide accurate oxygen saturation measurements apart from the other measurements (of other types) according to the multifunctional nature of said add-sensor pressure cuffs. Multifunctionality concentrated in same pressure cuff is thus furthermore improved with said other features.

The non-invasive oxygen saturation sensor may be based on infrared LED technology or other type of probe technology or any combination thereof.

In add-sensor pressure cuffs of example, the air tube may include a plurality of electro-lines along whole length thereof, each of the electro-lines being configured to transmit electro-signals between the air-pressure cuff and the healthcare monitor.

In some configurations, the one or more non-invasive non-pressure-based sensors may comprise a non-invasive ElectroCardioGram, ECG, arrangement including non-invasive ECG sensors each connectable with the healthcare monitor through associated electro-line in the plurality of electro-lines. Such non-invasive ECG sensors may be distributed in/on/at the inflatable bag in such a manner that, when (the inflatable bag is) installed in the predefined disposition, the non-invasive ECG sensors result positioned onto blood vessels of the patient's limb that are known to provide optimum or useful ECG signals according to cardiology literature. Since these optimal vessel positions for accurate ECG performance are already known in medical side of the technical field(s) at hand, no details on this matter are provided herein.

In some exemplary add-sensor pressure cuffs, the ECG arrangement may further include a non-invasive ground connector connectable with the healthcare monitor through associated electro-line in the plurality of electro-lines. The non-invasive ground connector may be arranged in/on/at the inflatable bag in such a manner that, when (the inflatable bag is) installed in the predefined disposition, the non-invasive ground connector results exposed and therefore touchable by a patient's body part different from the patient's limb around which the inflatable bag is installed. This innovative feature regarding ground connector touchable by the patient his/herself enables add-sensor pressure cuffs to perform non-invasive ECGs reasonably accurately at patient's limb.

In add-sensor pressure cuffs according to examples, the inflatable bag may have non-invasive electrodes arranged in/on/at the inflatable bag in such a manner that, when (the inflatable bag is) installed in the predefined disposition, the non-invasive electrodes result placed onto a target muscle's nerve of the patient's limb. Each of the non-invasive electrodes may be connectable with the healthcare monitor through associated electro-line in the plurality of electro-lines. The electro-lines associated to the non-invasive electrodes may be configured to transmit electro-stimulation signals from the healthcare monitor to the non-invasive electrodes to electro-stimulate the target muscle's nerve of the patient's limb. Said electro-stimulation may be such that a muscular response by the target muscle is provoked in such a manner that pressure variations are induced in the inflatable bag. Such pressure variations may be or are to be used by the healthcare monitor to determine a medical condition of the patient depending on said induced air pressure variations. Said medical condition may be a neuromuscular condition such as, e.g., a neuromuscular blockade status of the patient.

The above features regarding measuring of medical conditions based on electro-stimulation of muscle's nerve at patient's limb and measuring muscle response due to said electro-stimulation further expand the range of measurement types provided by multifunctional add-sensor pressure cuffs. Multifunctionality concentrated in same air-pressure cuff is thus additionally improved with said other features.

In some implementations, add-sensor pressure cuffs may further comprise a memory unit and a controller module configured to operate the memory unit to store and/or update data in the memory unit, and/or to exchange data between the healthcare monitor and the memory unit through at least some of the electro-lines. Said data storable and/or updatable in the memory unit may include data generated at the air-pressure cuff itself or received from the healthcare monitor through at least some of the electro-lines. With these features regarding memory and controller, the wide range of different measurements that are obtainable with multifunctional (add-sensor) pressure cuffs may be stored in the cuff itself, updated if/when needed, transferred to healthcare monitor, etc. Multifunctionality of said add-sensor pressure cuffs is thus maximized not only from perspective of multifunctional measurements.

Add-sensor pressure cuffs may be similar to any of the memory (or mem-equipped) pressure cuffs or flex-connector pressure cuffs or air-pressure cuffs in interface medical systems disclosed herein or may have any of the features thereof.

Medical measuring systems (or simply measuring systems) may also be provided including any of the add-sensor pressure cuffs described herein and the healthcare monitor. Medical or surgical rooms including any of said medical measuring systems may also be provided. Medical or surgical vehicles including any of said medical measuring systems may be provided as well.

### BRIEF DESCRIPTION OF THE DRAWINGS

Non-limiting examples of the disclosure will be described in the following, with reference to the appended drawings, in which:
Figure 1 is a block diagram schematically illustrating interface medical systems according to examples.
Figure 2 is a block diagram schematically illustrating interface controllers according to examples, which are suitable for interface medical systems such as those of Figure 1.
Figure 3 is a flow chart schematically illustrating interface control methods according to examples, which are suitable for controlling interface medical systems such as those of Figure 1 and are implementable by interface controllers such as those of Figure 2.
Figure 4 is a block diagram schematically illustrating memory pressure cuffs according to examples.
Figure 5 is a flow chart schematically illustrating memory control methods according to examples, which are suitable for controlling memory pressure cuffs such as those of Figure 4.
Figures 6 and 7 are a block diagrams schematically illustrating flex-connector pressure cuffs according to examples.
Figure 8 is a block diagram schematically illustrating add-sensor pressure cuffs according to examples.

### DETAILED DESCRIPTION OF EXAMPLES

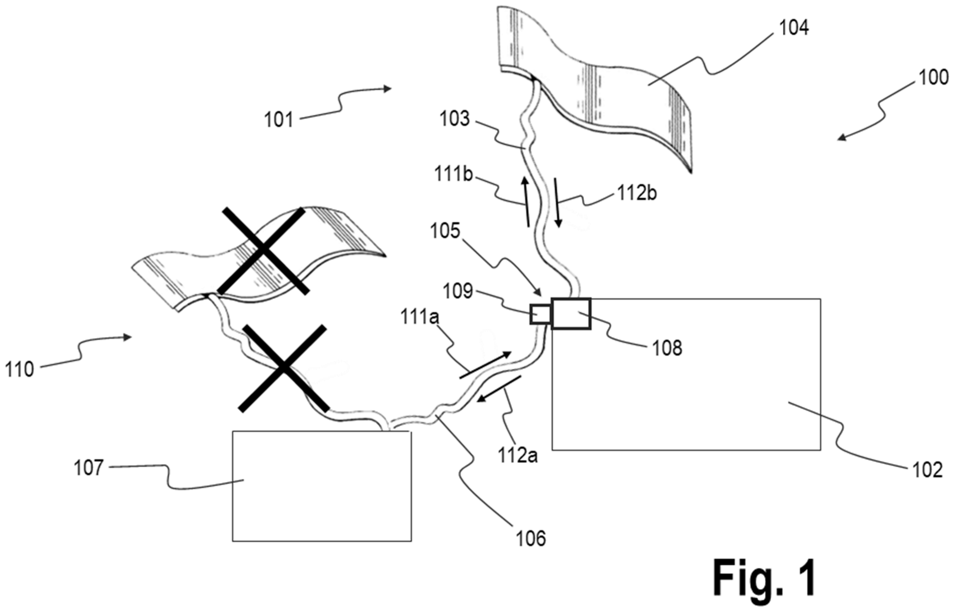

Figure 1 is a block diagram schematically illustrating interface medical systems 100, according to examples. As shown in the figure, interface medical systems 100 may include an air-pressure cuff 101 and a medical monitor 102. The air-pressure cuff 101 may have an inflatable bag 104 installable in predefined disposition around a patient's limb. The medical monitor 102 may be configured to perform non-invasive blood pressure measurement on the patient through the air-pressure cuff 101.

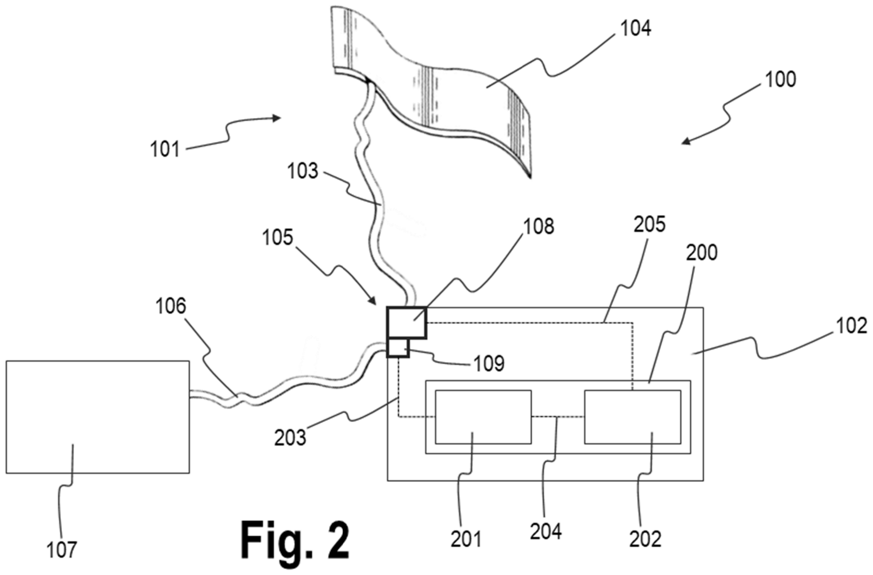

The medical monitor 102 may comprise an interface arrangement 105 configured to implement an air connection 106 between the medical monitor 102 and a healthcare monitor 107 different from the medical monitor 102, and to operate an air flow regulator 108 to keep the air connection 106 opened or closed. The interface arrangement 105 (or a controller thereof, as illustrated in Figure 2) may be configured to operate an air flow detector 109 to detect start of non-invasive blood pressure measurement from the healthcare monitor 107 and, in response to said detection, to operate the air flow regulator 108 to keep the air connection 106 opened. By keeping the air connection 106 opened, the started non-invasive blood pressure measurement is performed by the healthcare monitor 107 through the air-pressure cuff 101 of the medical system 100 instead of using an air-pressure cuff 110 directly associated to the healthcare monitor 107.

**It** is shown in the drawing that start of the non-invasive blood pressure measurement from healthcare monitor 107 may be detected by the detector 109 by sensing air flow 111a from healthcare monitor 107 and, furthermore, that said detection may trigger operation of the air flow regulator 108 to let the air flow pass through the interface arrangement 105 and continue in direction 111b towards inflatable bag 104. Similarly, it is also illustrated the reverse air flow due to air pressure changes in the inflatable bag 104 firstly following direction 112b towards interface arrangement 105, secondly passing through regulator 108 and thirdly continuing in direction 112a towards healthcare monitor 107.

The inflatable bag 104 may have non-invasive electrodes (not shown) electrically connected (or connectable) with the medical monitor 102 and arranged in/on/at the inflatable bag 104 in such a manner that, when the inflatable bag 104 is installed in the predefined disposition (around patient's limb), the non-invasive electrodes result/are placed onto target muscle's nerve of the patient's limb. The medical monitor 102 may be configured to electro-stimulate the target muscle's nerve through the non-invasive electrodes to provoke a muscular response by the target muscle to said electro-stimulation, and to determine a neuromuscular condition of the patient depending on air pressure variations in the inflatable bag 104 caused by said muscular response.

The air connection 106 implemented by the interface arrangement 105 may be a pneumatic air connection. The air flow regulator 108 may include a valve operable to open and close the air connection 106 such as, e.g., a three-way valve. The air flow detector 109 may include an air pressure sensor such as, e.g., a piezoresistive air pressure sensor, a capacitive air pressure sensor, a MEMS-based air pressure sensor or any combination thereof.

The interface arrangement 105 (or a controller thereof, as illustrated in Figure 2) may be configured to detect start of non-invasive blood pressure measurement from the healthcare monitor 107 depending on a pressure detected by the air pressure sensor 109. In particular, this detection may be performed depending on whether the detected pressure is above or below a predefined pressure threshold or within or outside a predefined pressure range. For example:
- If detected pressure above predefined pressure threshold, measurement may be assumed started. Otherwise, if detected pressure below predefined pressure threshold, measurement may be assumed not started.
- If detected pressure outside predefined pressure range, measurement may be assumed started. Otherwise, if detected pressure within predefined pressure range, measurement may be assumed not started.

The air flow regulator 108 may be operated by the interface arrangement 105 (or a controller thereof, as illustrated in Figure 2) to enable air flow in either direct mode o interfaced mode. Direct mode may mean that air flow between the inflatable bag 104 and the medical monitor 102 is enabled and air flow between the inflatable bag 104 and the healthcare monitor 107 is disabled. Interfaced mode may mean that air flow between the inflatable bag 104 and the medical monitor 102 is disabled and air flow between the inflatable bag 104 and the healthcare monitor 107 is enabled through the interface arrangement 105. Opening of the air connection 106 may be performed by operating the air flow regulator 108 to cause transition from the direct mode to the interfaced mode. Closing of the air connection 106 may be performed by operating the air flow regulator 108 to cause transition from the interfaced mode to the direct mode.

The interface arrangement 105 (or a controller thereof, as illustrated in Figure 2) may be configured to keep the air connection 106 opened (i.e., in the interfaced mode) by default to give priority to the healthcare monitor 107 over the medical monitor 102. In some implementations, maximum priority may be attributed to the healthcare monitor 107 by forcing the interfaced mode when start of measurement by the healthcare monitor 107 is detected, irrespective of whether measurement by the medical monitor 102 is in progress or not. Alternatively, if start of measurement by the healthcare monitor 107 is detected when measurement by the medical monitor 102 is in progress, transition from the direct mode to the interfaced mode may be performed upon completion of the measurement by the medical monitor 102. In any case, transition from the direct mode to the interfaced mode may be performed every time a measurement by the medical monitor 102 terminates, in order to return the air connection 106 to its default opened state.

Figure 2 is a block diagram schematically illustrating interface controllers 200 suitable for interface medical systems such as those of Figure 1, according to examples. Number references indicating same elements or similar as elements of Figure 1 have been kept in Figure 2. As shown, interface controllers 200 may include a detector module 201 and an operator module 202. The detector module 201 may be configured to operate, through connection 203, the air flow detector 109 to detect start of non-invasive blood pressure measurement from the healthcare monitor 107, in which case, the detector module 201 may signal, through connection 204, said detection to the operator module 202. Then, the operator module 202 may be configured to operate, through connection 205, the air flow regulator 108 to keep the air connection 106 opened in such a manner that the started non-invasive blood pressure measurement is performed by the healthcare monitor 107 through the air-pressure cuff 101 of the medical system 100 instead of using an air-pressure cuff directly associated to the healthcare monitor 107.

Operation of the air flow detector 109 may include receiving by the detector module 201 through connection 203 a signal indicating detection of air pressure from the healthcare monitor 107. Then, if said detected pressure is high enough to denote start of non-invasive blood pressure measurement by/from the healthcare monitor 107, the detector module 201 may signal, through connection 204, said condition to the operator module 202. In this case, the operator module 202 may operate or instruct, through connection 205, the air flow regulator 108 to keep the air connection 106 opened, in such a manner that use of the pressure cuff 101 is enabled to the healthcare monitor 107 and disabled to the medical monitor 102.

In the particular examples of Figure 2, the interface controller 200 is shown external to the interface arrangement 105 and internal to the medical monitor 102. However, any relative disposition between said components is possible. For example, the interface controller 200 may be internal to the interface arrangement 105 or external to both the medical monitor 102 and the interface arrangement 105. Proper connections may be included between the different components depending on the disposition chosen.

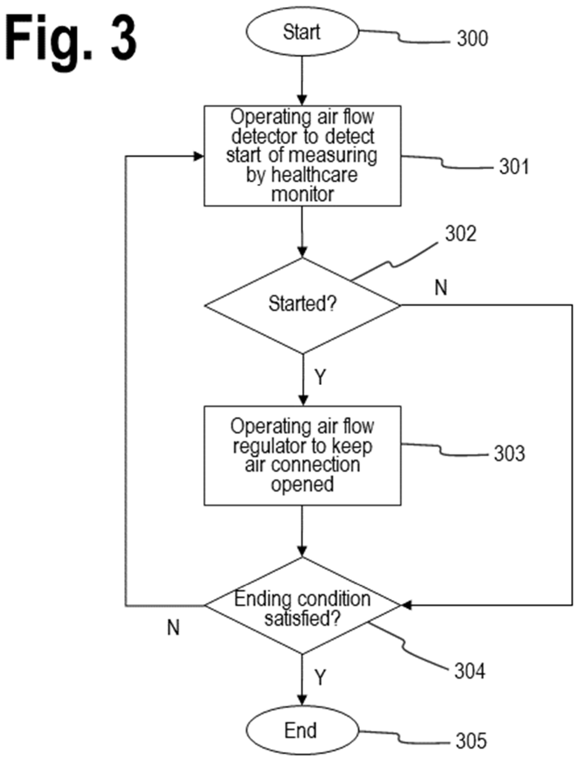

Figure 3 is a flow chart schematically illustrating interface control methods according to examples, which are suitable for or performable by interface medical systems such as those of Figure 1 and/or implementable by/through interface controllers such as those of Figure 2. As shown in this figure, interface control methods may be initiated (e.g., at block 300) upon detection of a starting condition such as, e.g., a user request to start the method. Since interface control methods according to Figure 3 are performable by/at interface medical systems according to Figure 1, or by/at interface controllers according to Figure 2, number references from Figures 1 and 2 may be reused in following description of Figure 3.

Interface control methods may further include operating (e.g., at method-block 301) the air flow detector 109 to detect, or not, start of non-invasive blood pressure measurement by/from the healthcare monitor 107 and transitioning (e.g., at method-block 302) to one or another method-block depending on whether detection has been effected or not. If detection has been effected Y, the method may continue to block 303 to operate the air flow regulator 108 accordingly and, otherwise N, to block 304 to determine whether execution of the method must terminate. This detection functionality implementable at method-blocks 301 and 302 is performable by interface arrangement 105 (or controller thereof) according to Figure 1 and/or by detector module 201 according to Figure 2. Hence, functional details and considerations explained about said detection function with reference to said figures may be similarly attributed or attributable to method-blocks 301 and 302.

Interface control methods may still further include operating (e.g., at method-block 303) the air flow regulator 108 to keep the air connection 106 opened, i.e., in the interfaced mode, in such a manner that use of the pressure cuff 101 is enabled to the healthcare monitor 107 and disabled to the medical monitor 102, and transitioning (e.g., at method-block 304) to one or another method-block depending on whether ending condition is satisfied. Ending condition may be or include, e.g., a user request to terminate execution of the method. Upon satisfaction of ending condition Y, transition to method-block 305 may be performed to terminate execution of the method. Otherwise N, the method may loop back to previous method-block 301 to perform a new iteration of the method to detect new start of measurement by the healthcare monitor 107 and proceed accordingly, as explained with respect to blocks 301 - 304.

Extensions or alternatives to methods according to Figure 3 are possible based on functional principles regarding, e.g., interfaced mode by default, transition between direct mode and interfaced mode depending on priority or priorities defined, etc. described with reference to Figures 1 and 2.

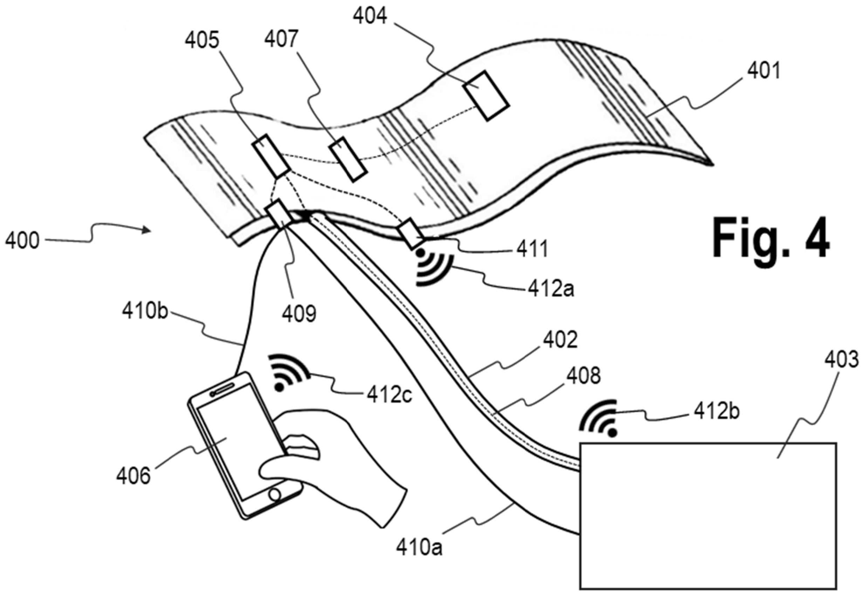

Figure 4 is a block diagram schematically illustrating memory pressure cuffs 400 according to examples. As shown, memory pressure cuffs 400 may comprise an inflatable bag 401, a connector arrangement with air tube 402, a memory unit 404, a data-comm (or data-communication) unit 405 and a controller module 407. The inflatable bag 401 may be installable in predefined disposition around patient's limb and to be inflated to experience air pressure variations in its inside due to interaction with the patient's limb. The air tube 402 may be configured to transmit air from a medical monitor 403 to the inflatable bag 401 to inflate it and to transmit air pressure variations from the inflatable bag 401 to the medical monitor 403. Said air pressure variations may be used by the medical monitor 403 to non-invasively measure patient's blood pressure depending thereon. The data-comm unit 405 may be configured to establish a data connection between the medical monitor 403 (or another type of computing device 406) and the memory unit 404. The controller module 407 may be configured to operate the data-comm unit 405 to establish the data connection and to operate the memory unit 404 to exchange data between the medical monitor 403 (or the other type of computing device 406) and the memory 404 unit through the established data connection.

In the particular examples of Figure 4, a smartphone 406 is shown as the other type of computing device 406 but it may be any device with computing capabilities such as, e.g., a laptop, a tablet, a desktop computer, any computer-based medical monitor, etc. Establishment of the data connection may include, e.g., detection of the medical monitor 403 to exchange data with, pre-setting of connection parameters to make the data connection available, pre-dialogue with the medical monitor to confirm it is available for data connection, etc. or any known action aimed at enabling data connection.

The connector arrangement may comprise a wired data connection 408 (illustrated as dashed line) integrated with or within the air tube 402. The wired data connection 408 may be operable or operated by the controller module 407 to exchange data between the medical monitor 403 and the memory unit 404.

The data-comm unit 405 may comprise a physical connection unit 409 and/or a wireless unit 411. The physical connection unit 409 may be operable or operated by the controller module 407 to establish physical data connection 410a-b with the medical monitor 403 (or smartphone 406) and to exchange data through said physical data connection 410a-b between the medical monitor 403 (or smartphone 406) and the memory unit 404. The wireless unit 411 may be operable or operated by the controller module 407 to establish wireless data connection 412a-b-c with the medical monitor 403 (or smartphone 406) and to exchange data through said wireless data connection 412a-b-c between the medical monitor 403 (or smartphone 406) and the memory unit 404.

Establishment of the physical data connection 410a-b or of the wireless data connection 412a-b-c may include any preliminary action or setting aimed at or necessary for implementing data connection (either physically 410a-b or wirelessly 412a-b-c) known in the prior art.

The wireless data connection 412a-b-c implementable or implemented through/by the wireless unit 411 may be a Radio Frequency connection, an NFC connection, a wifi connection, a bluetooth connection, a RFID connection, any other type of wireless data connection or any combination thereof.

The inflatable bag 401 may have non-invasive electrodes (not shown) that may be placed in/on/at the inflatable bag 401 in such a manner that, when (the inflatable bag 401 is) installed in the predefined disposition, the non-invasive electrodes result positioned onto a target muscle's nerve of the patient's limb. The connector arrangement may further include an electro-connection (not shown) configured to transmit electro-stimulation signals from the medical monitor 403 to the non-invasive electrodes to electro-stimulate the target muscle's nerve of the patient's limb. Said electro-stimulation may be such that muscular response by the target muscle is provoked and said muscular response causes air pressure variations in the inflatable bag 401 to be used by the medical monitor 403 to determine a medical condition of the patient depending on said air pressure variations. Such a medical condition of the patient determinable by the medical monitor may be a neuromuscular condition such as, e.g., a neuromuscular blockade status of the patient.

The memory unit 404 may permanently store invariable data of the air-pressure cuff 400 such as, e.g., manufacturing lot, or serial number, or manufacturer, or manufacturing date, or maximum number of uses, or expiration date, or indicator of disposable cuff, or useful life of the cuff, or electrodes impedance (if any), or any combination thereof.

The controller module 407 may be configured to update variable data of the air-pressure cuff 400 in the memory unit 404 (automatically or under request) such as, e.g., number of performed uses, or remaining useful life, or ID of patient is using the cuff, or ID of last patient used the cuff, or any combination thereof.

The controller module 407 may be configured to store and/or update patient-related data in the memory unit 404 such as, e.g., data on (a number of) last arterial pressure measurements performed, or data on (a number of) last neuromuscular transmission measurements performed, or allergies, or clinical history data, or current medication, or past medication, or patient's route through hospital between medical areas with dates and times, or any combination thereof. Said storable and/or updatable patient-related data may have been either obtained locally at the air-pressure cuff 400 or received from the medical monitor 403 (or smartphone 406) through data connection implemented by/through the data-comm unit 405.

The controller module 407 may be configured to dump data stored in the memory unit 404 to the medical monitor 403 (or smartphone 406) upon detection of a cuff-to-monitor dump request or upon detection of effective connection between the air-pressure cuff 400 and the medical monitor 403 (or smartphone 406). The controller module may be configured to select such data to be dumped by patient's ID.

The controller module 407 may be configured to dump data stored in the medical monitor 403 (or smartphone 406) to the memory unit 404 upon detection of monitor-to-cuff dump request or upon detection of effective connection between the air-pressure cuff 400 and the medical monitor 403 (or smartphone 406). The controller module may be configured to select such data to be dumped by patient's ID.

**In** the particular examples illustrated, the memory unit 404, data-comm unit 405 and controller module 407 are shown arranged in/on/at the inflatable bag 401 but, alternatively, said units 404, 405, 407 may be placed on/around the air tube 408 or in/on/at a coupling region between the bag 401 and tube 408. When placed in/on/at the inflatable bag 401, these units 404, 405, 407 may be attached to a surface of the inflatable bag 401 that does not contact the patient's limb when the bag 401 is in the predefined disposition. Irrespective of which parts of the cuff 400 the units 404, 405, 407 are attached or attachable to, said attachment may be removable. The units 404, 405, 407 may be integrated into same physical unit that may be removably attachable to the cuff 400, such that removal and attachment thereof is facilitated. Such a removable attachment may be, for example, click-based or Velcro-based or the like.

Pressure measuring systems may also be provided including any of the memory pressure cuffs 400 described herein and the medical monitor 403 (or the other type of computing device 406). Medical or surgical rooms including any of said pressure measuring systems may also be provided. Medical or surgical vehicles including any of said pressure measuring systems may be provided as well.

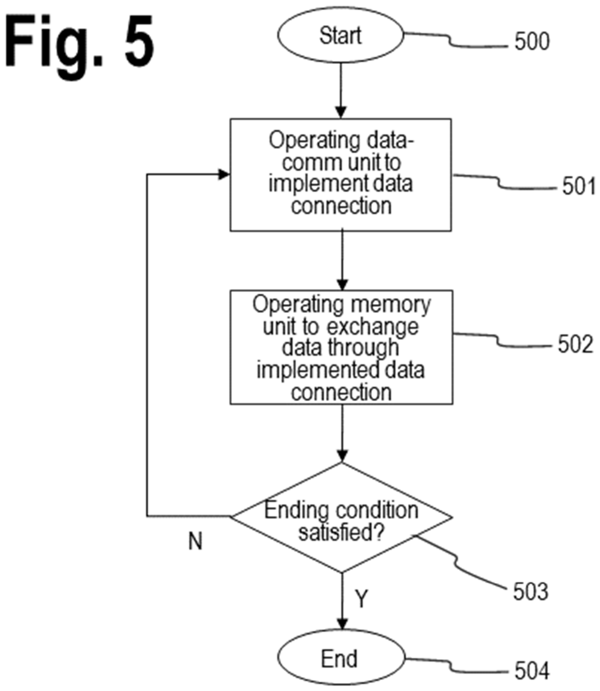

Figure 5 is a flow chart schematically illustrating memory control methods according to examples, which are suitable for controlling memory pressure cuffs such as those of Figure 4. As illustrated, memory control methods may be initiated (e.g., at block 500) upon detection of a starting condition such as, e.g., a user request to start the method. Since memory control methods according to Figure 5 are performable by/at memory pressure cuffs according to Figure 4 number references from said figure may be reused in following description of Figure 5.

Memory control methods may further include operating (e.g., at method-block 501), by the controller module 407, the data-comm unit 405 to implement data connection between the medical monitor 403 or the other computing device 406 and the memory unit 404. Since this data-connection functionality implementable at method-block 501 is performable by controller module 407 according to Figure 4, functional details and considerations explained about said data-connection function with reference to Figure 4 may be similarly attributed or attributable to method-block 501.

Memory control methods may still further include operating (e.g., at method-block 502), by the controller module 407, the memory unit 404 to exchange data between the medical monitor 403 or the other computing device 406 and the memory unit 404 through the implemented data connection. Since this data-exchange functionality implementable at method-block 502 is performable by controller module 407 according to Figure 4, functional details and considerations explained about said data-exchange function with reference to Figure 4 may be similarly attributed or attributable to method-block 502.

Memory control methods may additionally include (e.g., at decision block 503) verifying whether an ending condition occurs or is satisfied. In case of positive or true result (Y) of said verification, memory control method may proceed to method-block 504 to terminate execution of the memory control method. Otherwise (N), memory control method may loop back to previous method-block 501 to perform a new iteration to, e.g., perform new exchange of data as described before regarding combination of data-connection function 501 and data-exchange function 502. Ending condition may include, e.g., a user request to terminate the method.

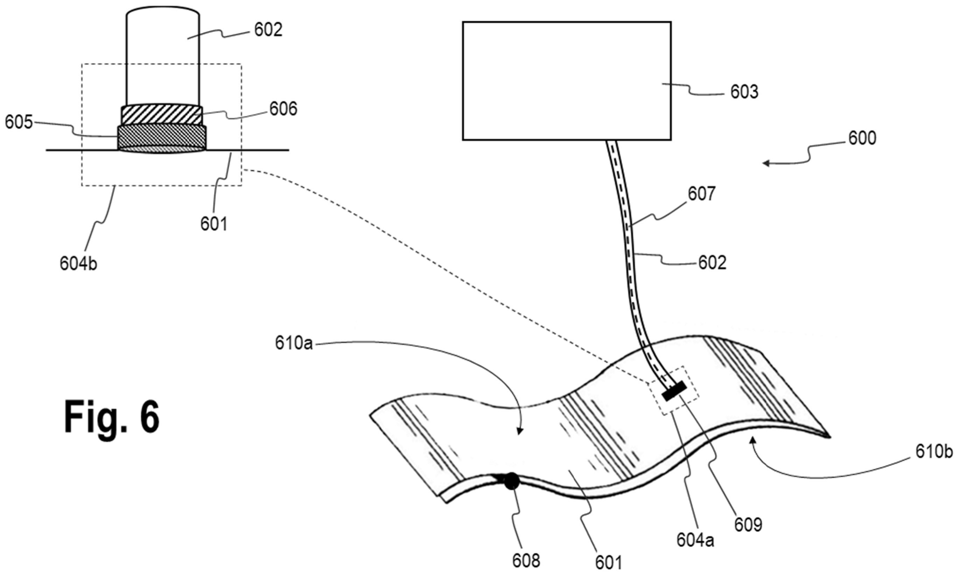

Figure 6 is a block diagram schematically illustrating flex-connector pressure cuffs 600 according to examples. As shown, flex-connector pressure cuffs may comprise an inflatable bag 601 and an air tube 602 connected with each other through coupling assembly 604a, the inflatable bag 601 being arrangeable in predefined disposition around a patient's limb. Such a coupling assembly is shown twice, in a small view thereof 604a and in an enlarged view thereof 604b. The coupling assembly 604a is shown, in the particular examples illustrated, at a location 609 on a main side 610a of the inflatable bag 601 but it may be located at other parts of the inflatable bag 601. For example, the coupling assembly 604a may be located at a lateral side or place 608 of the inflatable bag 601. The inflatable bag 601 may be seen as having two main sides: first main side and second main side. First main side 610a may be the one that results exposed or visible when the inflatable bag 601 is installed in the predefined disposition. Second main side 610b may be the one that results non-exposed or non-visible when the inflatable bag 601 is in the predefined disposition because said second main side 610b is contacting or facing patient's limb.

The inflatable bag 601 is and to be inflated to experience air pressure variations in its inside due to interaction with the patient's limb. The air tube 602 may be configured to transmit air from a medical monitor 603 to the inflatable bag 601 to inflate it and to transmit air pressure variations from the inflatable bag 601 to the medical monitor 603. Such air pressure variations may be used by the medical monitor 603 to non-invasively measure patient's blood pressure depending on said air pressure variations. The air tube 602 may include at one end thereof a tube-side connector 606, and the inflatable bag 601 may include a bag-side connector 605. One of the bag-side and tube-side connectors 605, 606 is removably couplable with the other of the bag-side and tube-side connectors 605, 606 in tight-fitting or press-fitting insertable manner. In the particular examples depicted, the tube-side connector 606 is shown tight-fitting or press-fitting insertable into the bag-side connector 605, but the reverse situation is also possible.

One of the bag-side and tube-side connectors 605, 606 may be a male connector 606 and the other of the bag-side and tube-side connectors 605, 606 may be a female connector 605. In the particular examples illustrated, the tube-side connector 606 is shown as male coupling part and the bag-side connector 605 is shown as female coupling part, but the reverse situation is also possible.

The bag-side and tube-side connectors 605, 606 may be rounded-shaped in such a manner that, when coupled with each other, the bag-side and tube-side connectors 605, 606 are rotatable with respect to each other. In particular, the bag-side and tube-side connectors 605, 606 may be cylinder-shaped. In the particular examples drawn, the tube-side connector 606 is shown internally rotatable with respect to the bag-side connector 605, but the reverse situation is also possible.

The air tube may further include one or more electro-lines 607 along whole length of the air tube 602, each of the electro-lines 607 being configured to transmit electro-signals such as, e.g., electro-stimulation signals, data transmission signals, etc.

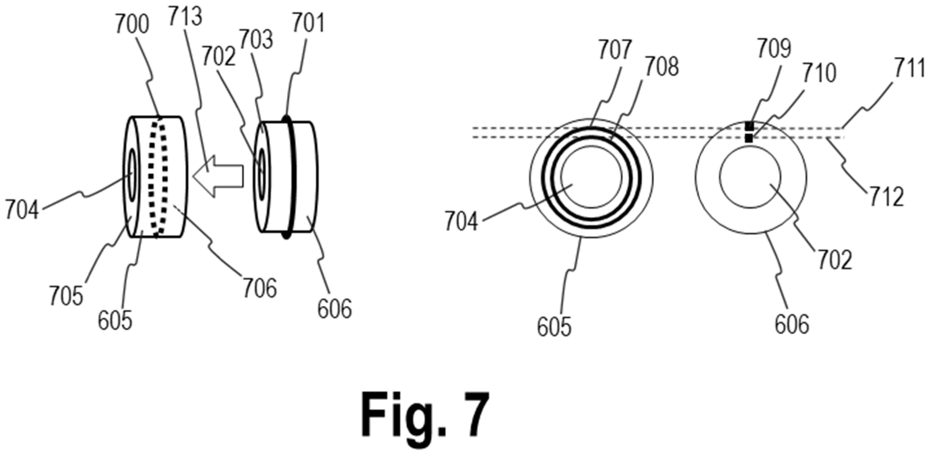

Figure 7 is a block diagram schematically illustrating in more detail bag-side and tube-side connectors 605, 606 according to examples. Since these bag-side and tube-side connectors 605, 606 are suitable for flex-connector pressure cuffs 600 according to Figure 6, number references from said figure may be reused in following description of Figure 7.

The bag-side and tube-side connectors 605, 606 may include a click-fit coupling mechanism which may be implemented in a diversity of manners. For example, one of the bag-side and tube-side connectors 605, 606 may have a protruding part 701 and the other of the bag-side and tube-side connectors 605, 606 may have a recessed part 700 in such a manner that, when the connectors 605, 606 are coupled with each other, the protruding part 701 click-fits the recessed part 700. In the particular examples depicted, the protruding part 701 is shown included in the tube-side connector 606 and the recessed part 700 is shown included in the bag-side connector 605, but the reverse situation is also possible.

The bag-side and tube-side connectors 605, 606 may implement, when coupled with each other, one or more electro-connections 707 - 710 between the inflatable bag 601 and the air tube 602. Each of the electro-lines 607 may end at the tube-side connector 606 with a connection pin or track 709, 710 arranged to, when the connectors 605, 606 are coupled with each other, electrically contact an associated connection pin or track 707, 708 at the bag-side connector 605.

Female connector 605 (at one of the bag and tube sides) may have hollow body part/volume 706 into which non-hollow body part/volume of the male connector 606 (at the other of the bag and tube sides) is insertable in tight-fitting or press-fitting manner. Connection pins or tracks 707, 708 in female connector 605 may be arranged on, e.g., flat surface 705 at bottom side of its hollow body part/volume 706, and connection pins or tracks 709, 710 in male connector 606 may be arranged on, e.g., flat surface 703 at front side of its non-hollow body part/volume. Said arrangement of pins and/or tracks 707 - 710 may be such that coupling of the connectors 605, 606 (with each other) causes pins or tracks 707, 708 in female connector 605 and pins or tracks 709, 710 in male connector 606 to come into electrical contact, respectively, as schematically suggested by or inferable from reference lines 711, 712.

In the particular examples depicted, the bag-side connector 605 is shown having connecting tracks 707, 708 and the tube-side connector 606 is shown having connecting pins (or pads) 709, 710, but any combination of tracks and pins is possible between the one and the other connector 605, 606. For reasons of simplicity, only two tracks 707, 708 and only two pins (or pads) 709, 710 are shown, but any other number of such connection elements 707 - 710 may be considered. For example, electro-connection may be based on connection tracks at both the bag-side and tube-side connectors 605, 606, or on connection tracks at the bag-side connector 605 and connection pins at the tube-side connector 606, or on connection pins at the bag-side connector 605 and connection tracks at the tube-side connector 606, etc.

**It** is exemplary shown in Figure 7 that connection tracks 707, 708 at one of the connectors 605, 606 may be concentrically arranged in such a manner that, when the connectors 605, 606 are coupled with each other, each of the connection tracks 707, 708 permanently contacts its associated connection pin or pins (or pads) 709, 710 at the other of the connectors 605, 606, irrespective of any rotational movement between the bag-side and tube-side connectors 605, 606. Theoretical reference line 711 illustrates alignment between track 707 and pin/pad 709 causing permanent electrical contact with each other. Theoretical reference line 712 illustrates alignment between track 708 and pin/pad 710 causing permanent electrical contact with each other.

**It** is further shown in the figure that the tube-side connector 606 may be (tight-fitting or press-fitting) insertable into the bag-side connector 605 following insertion direction 713, and extractable following reverse direction. It is also illustrated that bag-side connector 605 has hollow part 704 and non-hollow part 705 and that tube-side connector 606 has hollow part 702 and non-hollow part 703. Respective hollow parts 702, 704 may form tubed connection to let air pass through and respective non-hollow parts 703, 705 may have corresponding connection tracks or pins attached thereto forming the removable electro-connection(s).

**In** some flex-connector pressure cuffs, the inflatable bag 601 may have non-invasive electrodes (not shown) arranged in/on/at the inflatable bag 601 in such a manner that, when (the inflatable bag 601 is) installed in the predefined disposition, the non-invasive electrodes result placed onto a target muscle's nerve of the patient's limb, each of the non-invasive electrodes being electrically connected with respectively associated connection pin(s) or track(s) 707 - 710 at the bag-side connector 605. At least some of the electro-lines 607 may be configured to transmit electro-stimulation signals from the medical monitor 603 to the non-invasive electrodes to electro-stimulate the target muscle's nerve of the patient's limb. Said electro-stimulation may be such that muscular response by the target muscle is incited, thereby causing air pressure variations in the inflatable bag 601 to be used by the medical monitor 603 to determine a medical condition of the patient depending on said air pressure variations. Such a medical condition of the patient may be a neuromuscular condition such as, e.g., a neuromuscular blockade status of the patient.

Flex-connector pressure cuffs according to examples may further comprise a memory unit (not shown) and a controller module (not shown) configured to operate the memory unit to store and/or update data in the memory unit, and/or to exchange data between the medical monitor 603 and the memory unit through at least some of the electro-lines 607. Such data storable and/or updatable in the memory unit may be data generated at the flex-connector air-pressure cuff itself and/or received from the medical monitor 603 through at least some of the electro-lines 607.

Pressure measuring systems may also be provided including any of the flex-connector pressure cuffs 600 described herein and the medical monitor 603. Medical or surgical rooms including any of said pressure measuring systems may also be provided. Medical or surgical vehicles including any of said pressure measuring systems may be provided as well.

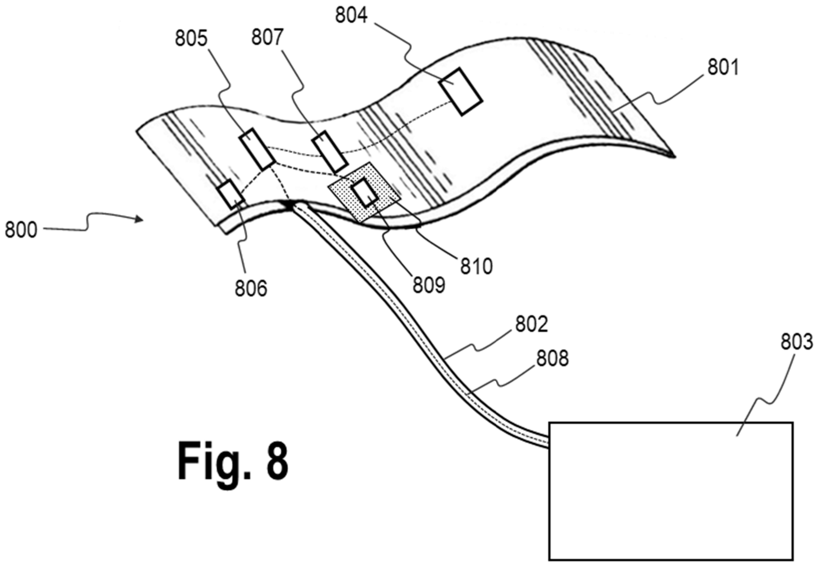

Figure 8 is a block diagram schematically illustrating add-sensor pressure cuffs 800 according to examples. As shown in the figure, add-sensor pressure cuffs 800 may comprise an inflatable bag 801, an air tube 802 and one or more non-invasive non-pressure-based sensors 804, 807, 809. The inflatable bag 801 may be configured to be installed in predefined disposition around a patient's limb and to be inflated to experience air pressure variations in its inside due to interaction with the patient's limb. The air tube 802 may be configured to transmit air from a healthcare monitor 803 to the inflatable bag 801 to inflate it and to transmit air pressure variations from the inflatable bag 801 to the healthcare monitor 803, such that patient's blood pressure may be non-invasively measured by the healthcare monitor 803 based on said air pressure variations. The non-invasive non-pressure-based sensors 804, 807, 809 may be arranged in/on/at the inflatable bag 801 in such a manner that, when (the inflatable bag 801 is) installed in the predefined disposition, the non-invasive non-pressure-based sensors 804, 807, 809 contact patient's skin.

In some implementations, the non-invasive non-pressure-based sensors 804, 807, 809 may include a non-invasive temperature sensor 809. Said non-invasive temperature sensor 809 may be arranged in/on/at the inflatable bag 801 in such a manner that, when (the inflatable bag 801 is) installed in the predefined disposition, the non-invasive temperature sensor 809 results positioned onto brachial artery of the patient's limb. A surface or region of the inflatable bag 801 in/on/at which the non-invasive temperature sensor 809 is arranged may include thermal insulating material 810 to avoid or minimize distortion of patient's temperature due to environmental conditions. Any known material that may maximize thermal insulation may be used to implement such an advantageous feature, which may make the add-sensor pressure cuff 800 optimal from perspective of temperature measuring. Since what materials and how may be used to implement this characteristic are known in the technical field at hand, no details on this question are provided in present disclosure.

The non-invasive temperature sensor 809 may be based on infrared LED technology or PT100 probe technology or K type probe technology or any combination thereof. Any known technology which permits implementing the non-invasive temperature sensor 809 reliably and under premisses of not excessive size in add-sensor pressure cuffs 800 may be used. Since technologies fulfilling said requirements are known in the involved technical field, no details on this question are provided in present disclosure.

Equipping air-pressure cuffs with temperature measuring capabilities in addition to other existing capabilities of, e.g., providing pressure-based (e.g., blood pressure) and/or oxygen saturation and/or ECG and/or neuromuscular measurements makes add-sensor pressure cuffs 800 much better than prior art air-pressure cuffs. Blood pressure and temperature (and optionally oxygen saturation, ECG heart-related condition, neuromuscular condition, etc.) may be measured simultaneously without the need of manipulating different devices nor disturbing the patient with different operations to measure different medical conditions.

In configurations according to examples, the non-invasive non-pressure-based sensors 804, 807, 809 include a non-invasive oxygen saturation sensor or pulse oximeter 807. Such a non-invasive oxygen saturation sensor 807 may be arranged in/on/at the inflatable bag 801 in such a manner that, when (the inflatable bag 801 is) installed in the predefined disposition, the non-invasive oxygen saturation sensor 807 results positioned with sensing surface or surfaces thereof fully contacting patient's skin. Any known manner suitable for implementing this feature may be used to do so. Since such manners suitable for fulfilling proposed requirements are known in the involved technical field, no details on said matter are provided in present disclosure.

The non-invasive oxygen saturation sensor 807 may be based on infrared LED technology or other type of probe technology or any combination thereof.

Equipping air-pressure cuffs with oxygen saturation measuring capabilities in addition to other existing capabilities of, e.g., providing pressure-based (e.g., blood pressure) and/or temperature and/or ECG and/or neuromuscular measurements makes add-sensor pressure cuffs 800 much better than prior art air-pressure cuffs. Blood pressure and oxygen saturation (and optionally temperature, ECG heart-related condition, neuromuscular condition, etc.) may be measured simultaneously without the need of manipulating different devices nor disturbing the patient with different operations to measure different medical conditions.

The air tube 802 may include electro-lines 808 along whole length of the air tube 802, each of the electro-lines 808 being configured to transmit electro-signals between the air-pressure cuff 800 and the healthcare monitor 803.

In exemplary configurations, the non-invasive non-pressure-based sensors 804, 807, 809 may comprise a non-invasive ElectroCardioGram, ECG, arrangement 804 including non-invasive ECG sensors 804 each connectable with the healthcare monitor 803 through associated electro-line of the electro-lines 808. The non-invasive ECG sensors 804 may be arranged or distributed in/on/at the inflatable bag 801 in such a manner that, when (the inflatable bag 801 is) installed in the predefined disposition, the non-invasive ECG sensors 804 result positioned onto blood vessels of the patient's limb that are known to provide optimum or useful ECG signals according to cardiology literature.

The ECG arrangement may further include a non-invasive ground connector (not shown) connectable with the healthcare monitor 803 through associated electro-line of the electro-lines 808. Such a non-invasive ground connector may be arranged in/on/at the inflatable bag 801 in such a manner that, when (the inflatable bag 801 is) installed in the predefined disposition, the non-invasive ground connector results exposed and therefore touchable by a patient's body part different from the patient's limb around which the inflatable bag 801 is installed.

Equipping air-pressure cuffs with ECG heart-related measuring capabilities in addition to other existing capabilities of, e.g., providing pressure-based (e.g., blood pressure) and/or temperature and/or Oxygen saturation and/or neuromuscular measurements makes add-sensor pressure cuffs 800 much better than prior art air-pressure cuffs. Blood pressure and ECG heart-related (and optionally temperature, Oxygen saturation condition, neuromuscular condition, etc.) may be measured simultaneously without the need of manipulating different devices nor disturbing the patient with different operations to measure different medical conditions.

According to some examples, the inflatable bag 801 may have non-invasive electrodes arranged in/on/at the inflatable bag 801 in such a manner that, when (the inflatable bag 801 is) installed in the predefined disposition, the non-invasive electrodes result placed onto a target muscle's nerve of the patient's limb, each of the non-invasive electrodes being connectable with the healthcare monitor 803 through associated electro-line of the electro-lines 808. The electro-lines 808 associated to the non-invasive electrodes may be configured to transmit electro-stimulation signals from the healthcare monitor 803 to the non-invasive electrodes to electro-stimulate the target muscle's nerve of the patient's limb. Said electro-stimulation may be such that a muscular response by the target muscle is provoked to make the inflatable bag 801 to experience air pressure variations in its inside, said pressure variations to be used or being usable by the healthcare monitor 803 to determine a medical condition of the patient depending on said air pressure variations. This medical condition of the patient determinable by the healthcare monitor 803 may be a neuromuscular condition such as, e.g., a neuromuscular blockade status of the patient.

Equipping air-pressure cuffs with neuromuscular measuring capabilities in addition to other existing capabilities of, e.g., providing pressure-based (e.g., blood pressure) and/or temperature and/or Oxygen saturation and/or ECG heart-related measurements makes add-sensor pressure cuffs 800 much better than prior art air-pressure cuffs. Blood pressure and neuromuscular (and optionally temperature, Oxygen saturation condition, ECG heart-related condition, etc.) may be measured simultaneously without the need of manipulating different devices nor disturbing the patient with different operations to measure different medical conditions.

Add-sensor pressure cuffs 800 may further comprise a controller module 805 and a memory unit 806 806. The controller 805 may be configured to operate the memory unit 806 to store and/or update data in the memory unit 806, and/or to exchange data between the healthcare monitor 803 and the memory unit 806 through at least some of the electro-lines 808. Data storable and/or updatable in the memory unit 806 may include data generated at the air-pressure cuff 800 itself or received from the healthcare monitor 803 through at least some of the electro-lines 808.

Equipping air-pressure cuffs with storing functionalities in addition to other existing capabilities of, e.g., providing pressure-based (e.g., blood pressure) and/or temperature and/or Oxygen saturation and/or ECG heart-related and/or neuromuscular measurements makes add-sensor pressure cuffs 800 much better than prior art air-pressure cuffs. The different measurement types of medical conditions combined with storing capabilities enable add-sensor pressure cuffs 800 to keep register or track locally (in the cuff 800 itself) of all said measurements and even transmitting them to medical/healthcare monitors 803 through suitable electro-lines connecting the cuff 800 with the medical/healthcare monitor 803.

Medical measuring systems may be also provided including any of the add-sensor pressure cuffs 800 disclosed herein and the healthcare monitor 803. Medical or surgical rooms including any of said medical measuring systems may be provided as well. Medical or surgical vehicles including any of said medical measuring systems may be additionally provided.

In relation to all or part of the air-pressure cuffs described herein, i.e., those included in interface medical systems and/or memory, flex-connector and/or add-sensor pressure cuffs according to present disclosure, following configurational aspects are noted.

In a first configurational aspect, the predefined disposition according to which inflatable bag is to be installed around patient's limb may be indicated through marks in/on the inflatable bag to assist or guide its operator to arrange it correctly in the predefined disposition. These marks may vary from one cuff to another depending on configurational parameters such as inflatable bag's size, shape, expandability, material, etc. What marks to be included and their arrangement in/on the inflatable bag to guide the operator to achieve the predefined disposition is a well-known issue or matter in the technical field of air-pressure cuffs, so no details thereon are provided in present disclosure. In fact, many simple non-multifunctional prior art pressure cuffs normally have such a kind of marks or indications suitably arranged, which is very well-known by the skilled person regarding what marks and where and how to include in/on the inflatable bag.

In a second configurational aspect, any sensor or electrode or any component arranged in/on the inflatable bag that need to contact patient's skin to operate correctly may be attached to a surface of the inflatable bag contacting the patient's limb/skin when the bag is in the predefined disposition. It is very well-known that an inflatable bag of any known air-pressure cuff arranged in its operational disposition (predefined disposition herein) results arranged with bag's first surface contacting patient's skin and bag's second surface not contacting patient's skin. This second surface is the one that results exposed and therefore visible while the first surface is the one that results not visible because it is hidden due its full or almost full contact with patient's skin. Thus, for example, implementation of the feature regarding non-invasive oxygen saturation sensor in/on/at add-sensor pressure cuffs inflatable bag with one or more sensing surfaces of the sensor fully contacting patient's skin is known and practicable by the skilled person depending on configurational parameters of the cuff and taking into account the predefined (or operational) disposition according to which the inflatable bag is to be arranged.

In a third configurational aspect, air-pressure cuffs disclosed herein have been described including an air tube configured to transmit air from medical/healthcare monitor to inflatable bag to inflate it (first air path) and to transmit air pressure variations from inflatable bag to medical monitor (second air path). It is noted that said air tube may be a single-way tube implementing both first and second air paths through same air way or a two-ways tube with one of the air ways implementing the first air path and the other of the air ways implementing the second air path. Also, air-pressure cuffs according to present disclosure may implement first and second air paths with one air tube for each of said air paths. It is understood that air tubes disclosed herein may be of any of said types: single-way tubes, two-ways tubes or double air tubes. Features described as being included in any of the air-pressure cuffs disclosed herein potentially affected or influenced by the type of air tube of the air-pressure cuff will be interpreted according to customary practice of the skilled person. Thus, for example, valve described as included or includable in interface medical systems will be two valves in the case of double air tubes, one valve for each of the air tubes in the double air tube. This is an example of implementation detail very well-known by the skilled person and therefore whose description is not required in a patent application since, otherwise, patents would be interminable.

Throughout this disclosure reference is made to neuromuscular-related aspects and features such as, e.g., neuromuscular functions, neuromuscular detection functionalities, neuromuscular condition, neuromuscular blockade state, neuromuscular-related measurements, neuromuscular transmission measurements, etc. For reasons of completeness, it is noted that details on at least some of these neuromuscular-related aspects and features can be found in EP application with publication number EP4241667A2.

As used herein, the term "module" may be understood to refer to software, firmware, hardware and/or various combinations thereof. It is noted that the modules are exemplary. The modules may be combined, integrated, separated, and/or duplicated to support various applications. Also, a function described herein as being performed by a particular module may be performed by one or more other modules and/or by one or more other devices instead of or in addition to the function performed by the described particular module.

The modules may be implemented across multiple devices, associated or linked to corresponding systems or controllers or devices proposed herein, and/or to other components that may be local or remote to one another. Additionally, the modules may be moved from one device and added to another device, and/or may be included in both devices, associated to corresponding systems or controllers or devices proposed herein. Any software implementations may be tangibly embodied in one or more storage media, such as, e.g., a memory device, a floppy disk, a compact disk (CD), a digital versatile disk (DVD), or other devices that may store computer code.

Systems or controllers or devices according to present disclosure may be implemented by computing means, electronic means or a combination thereof. The computing means may be a set of instructions (e.g., a computer program) and then systems or controllers or devices may comprise a memory and a processor, embodying said set of instructions stored in the memory and executable by the processor. These instructions may comprise functionality or functionalities to execute corresponding control methods such as, e.g., the ones described in other parts of the disclosure.

In systems or controllers or devices implemented only by electronic means, a controller of the system may be, for example, a CPLD (Complex Programmable Logic Device), an FPGA (Field Programmable Gate Array) or an ASIC (Application-Specific Integrated Circuit).

In systems or controllers or devices that are a combination of electronic and computing means, the computing means may be a set of instructions (e.g., a computer program) and the electronic means may be any electronic circuit capable of implementing corresponding steps of control methods proposed herein.

The computer program(s) may be embodied in/on a storage medium (for example, a CD-ROM, a DVD, a USB drive, a computer memory or a read-only memory) or carried on a carrier signal (for example, on an electrical or optical carrier signal).

The computer program(s) may be in the form of source code, object code, a code intermediate source and object code such as in partially compiled form, or in any other form suitable for use in implementing control methods according to present disclosure. The carrier may be any entity or device capable of carrying the computer program(s).

For example, the carrier may comprise a storage medium, such as a ROM, for example a CD ROM or a semiconductor ROM, or a magnetic recording medium, for example a hard disk. Further, the carrier may be a transmissible carrier such as an electrical or optical signal, which may be conveyed via electrical or optical cable or by radio or other means.

When the computer program(s) is/are embodied in a signal that may be conveyed directly by a cable or other device or means, the carrier may be constituted by such cable or other device or means. Alternatively, the carrier may be an integrated circuit in which the computer program(s) is/are embedded, the integrated circuit being adapted for performing, or for use in the performance of, control methods proposed herein.

For the sake of completeness and in order to provide a clear and exhaustive disclosure, the following set of clauses is hereby provided. These clauses are to be considered as part of the present description and illustrate various implementations and aspects of the technical developments proposed herein.

Clause 1. Medical system comprising an air-pressure cuff and a medical monitor, the air-pressure cuff having an inflatable bag installable in predefined disposition around a patient's limb, and the medical monitor being configured to perform non-invasive blood pressure measurement on the patient through the air-pressure cuff; wherein
the medical monitor comprises an interface arrangement configured to implement an air connection between the medical monitor and a healthcare monitor different from the medical monitor, and to operate an air flow regulator to keep the air connection opened or closed; and wherein
the interface arrangement is configured to operate an air flow detector to detect start of non-invasive blood pressure measurement from the healthcare monitor and, in response to said detection, to operate the air flow regulator to keep the air connection opened in such a manner that the started non-invasive blood pressure measurement is performed by the healthcare monitor through the air-pressure cuff of the medical system instead of using an air-pressure cuff directly associated to the healthcare monitor.

Clause 2. Medical system according to Clause 1, wherein the inflatable bag has non-invasive electrodes electrically connected or connectable with the medical monitor and arranged in/on/at the inflatable bag in such a manner that, when installed in the predefined disposition, the non-invasive electrodes result placed onto a target muscle's nerve of the patient's limb, and
the medical monitor is configured to electro-stimulate the target muscle's nerve through the non-invasive electrodes to provoke a muscular response by the target muscle to said electro-stimulation, and to determine a neuromuscular condition of the patient depending on air pressure variations in the inflatable bag caused by said muscular response.

Clause 3. Medical system according to any of Clauses 1 or 2, wherein the interface arrangement is configured to operate the air flow regulator to keep the air connection opened by default to give priority to the healthcare monitor over the medical monitor.

Clause 4. Medical system according to any of Clauses 1 to 3, wherein the interface arrangement is configured to operate the air flow regulator to keep the air connection closed during performance of non-invasive blood pressure measurement by the medical monitor through its pressure cuff.

Clause 5. Medical system according to Clause 4, wherein the interface arrangement is configured to operate the air flow regulator to open the air connection upon completion of non-invasive blood pressure measurement by the medical monitor through its pressure cuff.

Clause 6. Medical system according to any of Clauses 4 or 5, wherein the interface arrangement is configured to operate the air flow regulator to open the air connection upon detection of start of non-invasive blood pressure measurement from the healthcare monitor, irrespective of whether non-invasive blood pressure measurement by the medical monitor is in progress or not.

Clause 7. Medical system according to any of Clauses 1 to 6, wherein the air connection implemented by the interface arrangement is a pneumatic air connection, and/or the air flow regulator includes a valve operable to open and close the air connection, and/or the air flow detector includes an air pressure sensor.

Clause 8. Medical system according to Clause 7, wherein the valve operable to open and close the air connection is a three-way valve.

Clause 9. Medical system according to any of Clauses 7 or 8, wherein the air pressure sensor includes a piezoresistive air pressure sensor, a capacitive air pressure sensor, a MEMS-based air pressure sensor or any combination thereof.

Clause 10. Medical system according to any of Clauses 7 to 9, wherein the interface arrangement is configured to detect start of non-invasive blood pressure measurement from the healthcare monitor through the air pressure sensor depending on whether a pressure detected by the air pressure sensor is above or below predefined pressure threshold or within or outside predefined pressure range.

Clause 11. Method of controlling a medical system comprising an air-pressure cuff and a medical monitor, the air-pressure cuff having an inflatable bag installable in predefined disposition around a patient's limb, and the medical monitor being configured to perform non-invasive blood pressure measurement on the patient through the air-pressure cuff; wherein
the medical monitor comprises an interface arrangement configured to implement an air connection between the medical monitor and a healthcare monitor different from the medical monitor, and to operate an air flow regulator to keep the air connection opened or closed; and wherein the method comprises
operating an air flow detector to detect start of non-invasive blood pressure measurement from the healthcare monitor and, in response to said detection,
operating the air flow regulator to keep the air connection opened in such a manner that the started non-invasive blood pressure measurement is performed by the healthcare monitor through the air-pressure cuff of the medical system instead of using an air-pressure cuff directly associated to the healthcare monitor.

Clause 12. Computer program comprising program instructions for causing a controller or computing system to perform a method according to Clause 11 of controlling a medical system.

Clause 13. Controller for controlling a medical system comprising an air-pressure cuff and a medical monitor, the air-pressure cuff having an inflatable bag installable in predefined disposition around a patient's limb, and the medical monitor being configured to perform non-invasive blood pressure measurement on the patient through the air-pressure cuff; wherein
the medical monitor comprises an interface arrangement configured to implement an air connection between the medical monitor and a healthcare monitor different from the medical monitor, and to operate an air flow regulator to keep the air connection opened or closed; and wherein the controller comprises
a detector module configured to operate an air flow detector to detect start of non-invasive blood pressure measurement from the healthcare monitor and, in response to said detection,
an operator module configured to operate the air flow regulator to keep the air connection opened in such a manner that the started non-invasive blood pressure measurement is performed by the healthcare monitor through the air-pressure cuff of the medical system instead of using an air-pressure cuff directly associated to the healthcare monitor.

Clause 14. Healthcare system comprising a medical system according to any of Clauses 1 to 10 and the healthcare monitor.

Clause 15. Medical or surgical room including a healthcare system according to Clause 14.

Clause 16. Medical or surgical vehicle including a healthcare system according to Clause 14.

Clause 17. Air-pressure cuff comprising
an inflatable bag configured to be installed in predefined disposition around a patient's limb and to be inflated to experience air pressure variations in its inside due to interaction with the patient's limb;
a connector arrangement including an air tube configured to transmit air from a medical monitor to the inflatable bag to inflate it and to transmit air pressure variations from the inflatable bag to the medical monitor, in such a manner that patient's blood pressure is non-invasively measurable by the medical monitor depending on said air pressure variations;
a memory unit configured to store data;
a data-comm unit configured to implement a data connection between the medical monitor, or another type of computing device, and the memory unit; and
a controller module configured to operate the data-comm unit to implement the data connection and to operate the memory unit to exchange data between the medical monitor, or the other type of computing device, and the memory unit through the implemented data connection.

Clause 18. Air-pressure cuff according to Clause 17, wherein the connector arrangement further includes a wired connection integrated with the air tube, the wired connection being operable by the controller module to exchange data between the medical monitor and the memory unit.

Clause 19. Air-pressure cuff according to any of Clauses 17 or 18, further comprising a physical connection unit operable by the controller module to implement a physical connection with the medical monitor, or the other type of computing device, and to exchange data through said physical connection between the medical monitor, or the other type of computing device, and the memory unit.

Clause 20. Air-pressure cuff according to any of Clauses 17 to 19, further comprising a wireless unit operable by the controller module to implement a wireless connection with the medical monitor, or the other type of computing device, and to exchange data through said wireless connection between the medical monitor, or the other type of computing device, and the memory unit.

Clause 21. Air-pressure cuff according to Clause 20, wherein the wireless connection implementable through/by the wireless unit is a Radio Frequency connection, an NFC connection, a wifi connection, a bluetooth connection, a RFID connection, any other type of wireless connection or any combination thereof.

Clause 22. Medical system according to any of Clauses 17 to 21, wherein the inflatable bag has non-invasive electrodes arranged in/on/at the inflatable bag in such a manner that, when installed in the predefined disposition, the non-invasive electrodes result placed onto a target muscle's nerve of the patient's limb, and
the connector arrangement further includes an electro-connection configured to transmit electro-stimulation signals from the medical monitor to the non-invasive electrodes to electro-stimulate the target muscle's nerve of the patient's limb, in such a manner that
said electro-stimulation provokes a muscular response by the target muscle thereby causing air pressure variations in the inflatable bag to be used by the medical monitor to determine a medical condition of the patient depending on said air pressure variations.

Clause 23. Air-pressure cuff according to Clause 22, wherein the medical condition of the patient determinable by the medical monitor is a neuromuscular blockade status of the patient.

Clause 24. Air-pressure cuff according to any of Clauses 17 to 23, wherein the memory unit permanently stores manufacturing data of the air-pressure cuff.

Clause 25. Air-pressure cuff according to Clause 24, wherein the manufacturing data includes at least one of following data: manufacturing lot, serial number, manufacturer, manufacturing date, maximum number of uses, expiration date, indicator of disposable cuff, useful life of the cuff, electrodes impedance if any.

Clause 26. Air-pressure cuff according to any of Clauses 17 to 25, wherein the controller module is configured to automatically update variable data stored in the memory unit.

Clause 27. Air-pressure cuff according to Clause 26, wherein the updatable variable data includes at least one of following data: number of performed uses, remaining useful life, ID of patient is using the cuff, ID of last patient used the cuff.

Clause 28. Air-pressure cuff according to any of Clauses 17 to 27, wherein the controller module is configured to store and/or update patient-related data in the memory unit, either obtained locally at the air-pressure cuff or received from the medical monitor, or the other type of computing device.

Clause 29. Air-pressure cuff according to Clause 28, wherein the storable and/or updatable patient-related data includes at least one of following data: a number of last arterial pressure measurements, a number of last neuromuscular transmission measurements, allergies, clinical history data, current medication, past medication, patient's route through hospital between medical areas with dates and times.

Clause 30. Air-pressure cuff according to any of Clauses 17 to 29, wherein the controller module is configured to automatically dump data stored in the memory unit to the medical monitor, or the other type of computing device, upon detection of a cuff-to-monitor dump request or upon detection of data connection between the air-pressure cuff and the medical monitor, or the other type of computing device.

Clause 31. Air-pressure cuff according to any of Clauses 17 to 30, wherein the controller module is configured to automatically dump data stored in the medical monitor, or the other type of computing device, to the memory unit upon detection of monitor-to-cuff dump request or upon detection of connection between the air-pressure cuff and the medical monitor, or the other type of computing device.

Clause 32. Air-pressure cuff according to any of Clauses 30 or 31, wherein the controller module is configured to select the data to be dumped by patient's ID.

Clause 33. Pressure measuring system including an air-pressure cuff according to any of Clauses 17 to 32 and the medical monitor, or the other type of computing device.

Clause 34. Medical or surgical room including a pressure measuring system according to Clause 33.

Clause 35. Medical or surgical vehicle including a pressure measuring system according to Clause 33.

Clause 36. Method of controlling an air-pressure cuff comprising
an inflatable bag configured to be installed in predefined disposition around a patient's limb and to be inflated to experience air pressure variations in its inside due to interaction with the patient's limb,
a connector arrangement including an air tube configured to transmit air from a medical monitor to the inflatable bag to inflate it and to transmit air pressure variations from the inflatable bag to the medical monitor, in such a manner that patient's blood pressure is non-invasively measurable by the medical monitor depending on said air pressure variations, and
a memory unit, a data-comm unit and a controller module;
the method comprising
operating, by the controller module, the data-comm unit to implement a data connection between the medical monitor, or another type of computing device, and the memory unit; and
operating, by the controller module, the memory unit to exchange data between the medical monitor, or the other type of computing device, and the memory unit through the implemented data connection.

Clause 37. Computer program comprising program instructions for causing a controller or computing system to perform a method according to Clause 36 of controlling an air-pressure cuff.

Clause 38. Air-pressure cuff comprising
an inflatable bag configured to be installed in predefined disposition around a patient's limb and to be inflated to experience air pressure variations in its inside due to interaction with the patient's limb, the inflatable bag including a bag-side connector; and
an air tube configured to transmit air from a medical monitor to the inflatable bag to inflate it and to transmit air pressure variations from the inflatable bag to the medical monitor, in such a manner that patient's blood pressure is non-invasively measurable by the medical monitor depending on said air pressure variations, the air tube including at one end thereof a tube-side connector;
wherein one of the bag-side and tube-side connectors is couplable with the other of the bag-side and tube-side connectors in tight-fitting or press-fitting insertable manner.

Clause 39. Air-pressure cuff according to Clause 38, wherein one of the bag-side and tube-side connectors is a male connector and the other of the bag-side and tube-side connectors is a female connector.

Clause 40. Air-pressure cuff according to Clause 39, wherein the bag-side and tube-side connectors are rounded-shaped in such a manner that, when coupled, the bag-side and tube-side connectors are rotatable with respect to each other.

Clause 41. Air-pressure cuff according to Clause 40, wherein the bag-side and tube-side connectors are cylinder-shaped in such a manner that, when coupled, the bag-side and tube-side connectors are rotatable with respect to each other.

Clause 42. Air-pressure cuff according to any of Clauses 38 to 41, wherein the bag-side and tube-side connectors include a click-fit coupling mechanism.

Clause 43. Air-pressure cuff according to Clause 42, wherein the click-fit coupling mechanism is based on a protruding part at one of the bag-side and tube-side connectors and a recessed part at the other of the bag-side and tube-side connectors in such a manner that, when the connectors are coupled, the protruding part click-fits the recessed part.

Clause 44. Air-pressure cuff according to any of Clauses 38 to 43, wherein the air tube further includes one or more electro-lines along entire length of the air tube, each of the electro-lines being configured to transmit electro-signals.

Clause 45. Air-pressure cuff according to Clause 44, wherein the bag-side and tube-side connectors implement, when coupled, an electro-connection between the inflatable bag and the air tube based on that each of the electro-lines terminates at the tube-side connector with a connection pin or track arranged to, when the connectors are coupled, electrically contact an associated connection pin or track at the bag-side connector.

Clause 46. Air-pressure cuff according to Clause 45, wherein the bag-side and tube-side connectors are, when coupled, rotatable with respect to each other; wherein
the electro-connection is fully based on connection tracks at both the bag-side and tube-side connectors; and wherein
the connection tracks in each of the bag-side and tube-side connectors are concentrically arranged in such a manner that, when the connectors are coupled, each of the connection tracks at the bag-side connector permanently contacts its associated connection track at the tube-side connector, irrespective of any rotational movement between the bag-side and tube-side connectors.

Clause 47. Air-pressure cuff according to Clause 45, wherein the bag-side and tube-side connectors are, when coupled, rotatable with respect to each other; wherein
the electro-connection is based on connection tracks at the bag-side connector and connection pins at the tube-side connector; and wherein
the connection tracks at the bag-side connector are concentrically arranged in such a manner that, when the connectors are coupled, each of the connection tracks at the bag-side connector permanently contacts its associated connection pin or pins at the tube-side connector, irrespective of any rotational movement between the bag-side and tube-side connectors.

Clause 48. Air-pressure cuff according to Clause 45, wherein the bag-side and tube-side connectors are, when coupled, rotatable with respect to each other; wherein
the electro-connection is based on connection pins at the bag-side connector and connection tracks at the tube-side connector; and wherein
the connection tracks at the tube-side connector are concentrically arranged in such a manner that, when the connectors are coupled, each of the connection tracks at the tube-side connector permanently contacts its associated connection pin or pins at the bag-side connector, irrespective of any rotational movement between the bag-side and tube-side connectors.

Clause 49. Air-pressure cuff according to any of Clauses 45 to 48, wherein the inflatable bag has non-invasive electrodes arranged in/on/at the inflatable bag in such a manner that, when installed in the predefined disposition, the non-invasive electrodes result placed onto a target muscle's nerve of the patient's limb, each of the non-invasive electrodes being electrically connected with respectively associated connection pin or track at the bag-side connector; and wherein
at least some of the electro-lines are configured to transmit electro-stimulation signals from the medical monitor to the non-invasive electrodes to electro-stimulate the target muscle's nerve of the patient's limb, in such a manner that
said electro-stimulation provokes a muscular response by the target muscle thereby causing air pressure variations in the inflatable bag to be used by the medical monitor to determine a medical condition of the patient depending on said air pressure variations.

Clause 50. Air-pressure cuff according to Clause 49, wherein the medical condition of the patient determinable by the medical monitor is a neuromuscular blockade status of the patient.

Clause 51. Air-pressure cuff according to any of Clauses 45 to 50, further comprising a memory unit and a controller module configured to operate the memory unit to store and/or update data in the memory unit, and/or to exchange data between the medical monitor and the memory unit through at least some of the electro-lines.

Clause 52. Air-pressure cuff according to Clause 51, wherein the data storable and/or updatable in the memory unit is data generated at the air-pressure cuff itself or received from the medical monitor through at least some of the electro-lines.

Clause 53. Pressure measuring system including an air-pressure cuff according to any of Clauses 38 to 52 and the medical monitor.

Clause 54. Medical or surgical room including a pressure measuring system according to Clause 53.

Clause 55. Medical or surgical vehicle including a pressure measuring system according to Clause 53.

Clause 56. Air-pressure cuff comprising
an inflatable bag configured to be installed in predefined disposition around a patient's limb and to be inflated to experience air pressure variations in its inside due to interaction with the patient's limb;
an air tube configured to transmit air from a healthcare monitor to the inflatable bag to inflate it and to transmit air pressure variations from the inflatable bag to the healthcare monitor, in such a manner that patient's blood pressure is non-invasively measurable by the healthcare monitor depending on said air pressure variations; and
one or more non-invasive non-pressure-based sensors arranged in/on/at the inflatable bag in such a manner that, when installed in the predefined disposition, the one or more non-invasive non-pressure-based sensors contact patient's skin.

Clause 57. Air-pressure cuff according to Clause 56, wherein the one or more non-invasive non-pressure-based sensors include a non-invasive temperature sensor.

Clause 58. Air-pressure cuff according to Clause 57, wherein the non-invasive temperature sensor is arranged in/on/at the inflatable bag in such a manner that, when installed in the predefined disposition, the non-invasive temperature sensor results positioned onto brachial artery of the patient's limb.

Clause 59. Air-pressure cuff according to any of Clauses 57 or 58, wherein a surface of the inflatable bag in/on/at which the non-invasive temperature sensor is arranged includes thermal insulating material to avoid or minimize distortion of patient's temperature due to environmental conditions.

Clause 60. Air-pressure cuff according to any of Clauses 57 to 59, wherein the non-invasive temperature sensor is based on infrared LED technology or PT100 probe technology or K type probe technology or any combination thereof.

Clause 61. Air-pressure cuff according to any of Clauses 56 to 60, wherein the one or more non-invasive non-pressure-based sensors include a non-invasive oxygen saturation sensor or pulse oximeter.

Clause 62. Air-pressure cuff according to Clause 61, wherein the non-invasive oxygen saturation sensor is arranged in/on/at the inflatable bag in such a manner that, when installed in the predefined disposition, the non-invasive oxygen saturation sensor results positioned with sensing surface or surfaces thereof fully contacting patient's skin.

Clause 63. Air-pressure cuff according to any of Clauses 61 or 62, wherein the non-invasive oxygen saturation sensor is based on infrared LED technology or other type of probe technology or any combination thereof.

Clause 64. Air-pressure cuff according to any of Clauses 56 to 63, wherein the air tube includes electro-lines along entire length of the air tube, each of the electro-lines being configured to transmit electro-signals between the air-pressure cuff and the healthcare monitor.

Clause 65. Air-pressure cuff according to Clause 64, wherein the one or more non-invasive non-pressure-based sensors comprise a non-invasive ElectroCardioGram, ECG, arrangement including non-invasive ECG sensors each connectable with the healthcare monitor through associated electro-line of the electro-lines.

Clause 66. Air-pressure cuff according to Clause 65, wherein the non-invasive ECG sensors are distributed in/on/at the inflatable bag in such a manner that, when installed in the predefined disposition, the non-invasive ECG sensors result positioned onto blood vessels of the patient's limb that are known to provide optimum or useful ECG signals according to cardiology literature.

Clause 67. Air-pressure cuff according to any of Clauses 65 or 66, wherein the ECG arrangement further includes a non-invasive ground connector connectable with the healthcare monitor through associated electro-line of the electro-lines.

Clause 68. Air-pressure cuff according to Clause 67, wherein the non-invasive ground connector is arranged in/on/at the inflatable bag in such a manner that, when installed in the predefined disposition, the non-invasive ground connector results exposed and therefore touchable by a patient's body part/volume different from the patient's limb around which the inflatable bag is installed.

Clause 69. Air-pressure cuff according to any of Clauses 64 to 68, wherein the inflatable bag has non-invasive electrodes arranged in/on/at the inflatable bag in such a manner that, when installed in the predefined disposition, the non-invasive electrodes result placed onto a target muscle's nerve of the patient's limb, each of the non-invasive electrodes being connectable with the healthcare monitor through associated electro-line of the electro-lines; and wherein
the electro-lines associated to the non-invasive electrodes are configured to transmit electro-stimulation signals from the healthcare monitor to the non-invasive electrodes to electro-stimulate the target muscle's nerve of the patient's limb, in such a manner that
said electro-stimulation provokes a muscular response by the target muscle thereby causing air pressure variations in the inflatable bag to be used by the healthcare monitor to determine a medical condition of the patient depending on said air pressure variations.

Clause 70. Air-pressure cuff according to Clause 69, wherein the medical condition of the patient determinable by the healthcare monitor is a neuromuscular blockade status of the patient.

Clause 71. Air-pressure cuff according to any of Clauses 64 to 70, further comprising a memory unit and a controller module configured to operate the memory unit to store and/or update data in the memory unit, and/or to exchange data between the healthcare monitor and the memory unit through at least some of the electro-lines.

Clause 72. Air-pressure cuff according to Clause 71, wherein the data storable and/or updatable in the memory unit is data generated at the air-pressure cuff itself or received from the healthcare monitor through at least some of the electro-lines.

Clause 73. Medical measuring system including an air-pressure cuff according to any of Clauses 56 to 72 and the healthcare monitor.

Clause 74. Medical or surgical room including a medical measuring system according to Clause 73.

Clause 75. Medical or surgical vehicle including a medical measuring system according to Clause 73.

Although only a number of examples have been disclosed herein, other alternatives, modifications, uses and/or equivalents thereof are possible. Furthermore, all possible combinations of the described examples are also covered. Thus, the scope of the disclosure should not be limited by particular examples, but it should be determined only by a fair reading of the claims that follow.

## Claims

1. Air-pressure cuff comprising
an inflatable bag configured to be installed in predefined disposition around a patient's limb and to be inflated to experience air pressure variations in its inside due to interaction with the patient's limb;
an air tube configured to transmit air from a healthcare monitor to the inflatable bag to inflate it and to transmit air pressure variations from the inflatable bag to the healthcare monitor, in such a manner that patient's blood pressure is non-invasively measurable by the healthcare monitor depending on said air pressure variations; and
one or more non-invasive non-pressure-based sensors arranged in/on/at the inflatable bag in such a manner that, when installed in the predefined disposition, the one or more non-invasive non-pressure-based sensors contact patient's skin.

2. Air-pressure cuff according to claim 1, wherein the one or more non-invasive non-pressure-based sensors include a non-invasive temperature sensor.

3. Air-pressure cuff according to claim 2, wherein the non-invasive temperature sensor is arranged in/on/at the inflatable bag in such a manner that, when installed in the predefined disposition, the non-invasive temperature sensor results positioned onto brachial artery of the patient's limb.

4. Air-pressure cuff according to any of claims 2 or 3, wherein a surface of the inflatable bag in/on/at which the non-invasive temperature sensor is arranged includes thermal insulating material to avoid or minimize distortion of patient's temperature due to environmental conditions.

5. Air-pressure cuff according to any of claims 2 to 4, wherein the non-invasive temperature sensor is based on infrared LED technology or PT100 probe technology or K type probe technology or any combination thereof.

6. Air-pressure cuff according to any of claims 1 to 5, wherein the one or more non-invasive non-pressure-based sensors include a non-invasive oxygen saturation sensor or pulse oximeter.

7. Air-pressure cuff according to claim 6, wherein the non-invasive oxygen saturation sensor is arranged in/on/at the inflatable bag in such a manner that, when installed in the predefined disposition, the non-invasive oxygen saturation sensor results positioned with sensing surface or surfaces thereof fully contacting patient's skin.

8. Air-pressure cuff according to any of claims 6 or 7, wherein the non-invasive oxygen saturation sensor is based on infrared LED technology or other type of probe technology or any combination thereof.

9. Air-pressure cuff according to any of claims 1 to 8, wherein the one or more non-invasive non-pressure-based sensors comprise a non-invasive ElectroCardioGram, ECG, arrangement including non-invasive ECG sensors each connectable with the healthcare monitor through associated electro-line in the air tube.

10. Air-pressure cuff according to claim 9, wherein the non-invasive ECG sensors are distributed in/on/at the inflatable bag in such a manner that, when installed in the predefined disposition, the non-invasive ECG sensors result positioned onto blood vessels of the patient's limb that are known to provide optimum or useful ECG signals according to cardiology literature.

11. Air-pressure cuff according to any of claims 9 or 10, wherein the ECG arrangement further includes a non-invasive ground connector connectable with the healthcare monitor through associated electro-line in the air tube.

12. Air-pressure cuff according to any of claims 1 to **11,** wherein the inflatable bag has non-invasive electrodes arranged in/on/at the inflatable bag in such a manner that, when installed in the predefined disposition, the non-invasive electrodes result placed onto a target muscle's nerve of the patient's limb, each of the non-invasive electrodes being connectable with the healthcare monitor through associated electro-line in the air tube; and wherein
electro-lines associated to the non-invasive electrodes are configured to transmit electro-stimulation signals from the healthcare monitor to the non-invasive electrodes to electro-stimulate the target muscle's nerve of the patient's limb, in such a manner that
said electro-stimulation provokes a muscular response by the target muscle thereby causing air pressure variations in the inflatable bag to be used by the healthcare monitor to determine a medical condition of the patient depending on said air pressure variations.

13. Air-pressure cuff according to claim 12, wherein the medical condition of the patient determinable by the healthcare monitor is a neuromuscular blockade status of the patient.

14. Medical measuring system including an air-pressure cuff according to any of claims 1 to 13 and the healthcare monitor.

15. Medical or surgical room or medical or surgical vehicle including a medical measuring system according to claim 14.
